(19) Europäisches Patentamt
European Patent Office
Office européen des brevets

(11) EP 2 072 130 A1

(12) **EUROPEAN PATENT APPLICATION**
published in accordance with Art. 153(4) EPC

(43) Date of publication:
**24.06.2009 Bulletin 2009/26**

(21) Application number: **07829758.7**

(22) Date of filing: **05.10.2007**

(51) Int Cl.:
***B01J 35/10*** *(2006.01)* ***B01J 29/072*** *(2006.01)*

(86) International application number:
**PCT/JP2007/070023**

(87) International publication number:
**WO 2008/044780 (17.04.2008 Gazette 2008/16)**

(84) Designated Contracting States:
**BE DE ES**
Designated Extension States:
**AL BA HR MK RS**

(30) Priority: **06.10.2006 JP 2006274620**
**06.10.2006 JP 2006274621**

(71) Applicant: **Kao Corporation**
**Chuo-ku**
**Tokyo 103-8210 (JP)**

(72) Inventors:
- **MATSUI, Kunio**
**Haga-gun, Tochigi; 3213497 (JP)**
- **NISHIMURA, Toru**
**Wakayama-shi, Wakayama; 6408580 (JP)**
- **SATO, Masayasu**
**Ichikai-machi, Haga-gun, Tochigi; 3213497 (JP)**

(74) Representative: **HOFFMANN EITLE**
**Patent- und Rechtsanwälte**
**Arabellastrasse 4**
**81925 München (DE)**

(54) **FILMY CATALYST AND PROCESS FOR PRODUCTION OF THE SAME**

(57) The present invention provides a film catalyst having a catalyst layer containing a powdery catalyst and a binder and a substrate on which the catalyst layer is formed, wherein the catalyst layer has a pore size distribution with two or more different mode values as determined by mercury porosimetry. The present invention also provides a method for producing a film catalyst containing a catalyst layer and a substrate on which the layer is formed, containing: preparing a coating composition containing a powdery catalyst, a binder and a solvent; and forming the catalyst layer on the substrate with the coating composition to give a catalyst intermediate, wherein the resultant film catalyst satisfies the formula (2-I) :

$$0.55\ T_1 < t_1 < 2\ T_1 \quad (2\text{-I})$$

wherein
$T_1$: the maximum mode value of the pore size distribution of the powdery catalyst itself used as a raw material contained in the catalyst layer of the film catalyst (as determined by mercury porosimetry) and
$t_1$: the maximum mode value of the pore size distribution of the catalyst layer of the film catalyst (as determined by mercury porosimetry).

Fig. 1

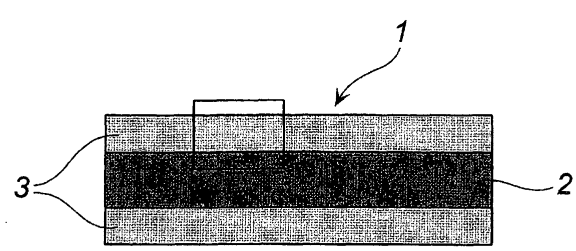


EP 2 072 130 A1

## Description

Field of the Invention

The present invention relates to a film catalyst suitably used as a catalyst for gas-liquid reaction, and to a process for producing the same.

Background of the Invention

In chemical industry, a wide variety of chemical processes using solid catalysts have been used. In these processes, many of catalysts used are powdery catalysts in the form of fine particle, and directly added to a reaction tank for reaction. In the reaction process using the powdery catalyst, techniques such as stirring for effectively mixing a substance to be reacted with the catalyst are required. The process also has other problem that after reaction ended, removal of the catalyst from a product must be conduct, which requires complicated facility and operations. Further, to a system of the process is added a large amount of a filtering aid. A filtration period is long since the catalyst is in the form of particle, and the filtering aid adsorbs a many part of the reaction product to decrease a yield of reaction.
Use of a porous carrier such as zeolite for carrying the powdery catalyst to increase a particle size of the powder may improve filtration properties of the fine particle powder and shorten a filtration period. In this case, the problems of complicated facility and operations and need for a large amount of filtering aid are still remaining to be solved.
There is a process, in contrast to these processes, which does not require an operation of mixing a catalyst such as by stirring and not require filtration separation of the catalyst, including an immobilized bed method. WO 2005/035122 discloses a film catalyst, which is a solid catalyst in the new form used in an immobilized method. The film catalyst having a thickness of not more than 100 μm is used for production of an amine, and enables to produce a tertiary amine in good reaction selectivity.

Summary of the Invention

The present invention provides a film catalyst, containing a substrate and a catalyst layer, formed on the substrate, containing a powdery catalyst and a binder and having a pore size distribution with two or more different mode values as determined by mercury porosimetry. (hereinafter, also referred to as the first invention).
The present invention provides a method for producing a film catalyst containing a substrate and a catalyst layer formed on the substrate, including steps:

preparing a coating composition containing a powdery catalyst, a binder and a solvent; and
forming the catalyst layer on the substrate with the coating composition to obtain a catalyst intermediate,

wherein the resultant film catalyst satisfies the formula (2-I):

$$0.55\ T_1 < t_1 < 2\ T_1 \quad (2\text{-I})$$

wherein
$T_1$: the maxim mode value of a pore size distribution of the powdery catalyst itself, used as a raw material contained in the catalyst layer of the film catalyst, as determined by mercury porosimetry and
$t_1$: the maxim mode value of a pore size distribution of the catalyst layer of the film catalyst, as determined by mercury porosimetry.
The present invention provides use of the film catalyst or a film catalyst obtained by the method for production as a catalyst for a gas-liquid reaction or a method for conducting a gas-liquid reaction with the film catalyst or a film catalyst obtained by the method for production.

Detailed Description of the Invention

The film catalyst disclosed in WO 2005/035122 is an excellent invention as it has good catalytic activity and is reduced in a falling amount of the catalyst.
The present invention improves the invention disclosed in WO 2005/035122, and provides a film catalyst (the first invention) having higher reactivity and more reduced in a falling amount of the catalyst and a method for producing the same (the second invention).

The first invention includes the following preferred embodiments:

the film catalyst having two or more different mode values as determined by mercury porosimetry, wherein the maximum mode value $t_1$ and a mode value $t_2$ different from the mode value $t_1$ satisfy a relationship represented by the formula (1-I):

$$t_2 \leq 0.2 \times t_1 \quad (1\text{-I});$$

the film catalyst having the mode value $t_1$ of 20 nm to 250 μm;
the film catalyst having the mode value $t_2$ of 4 to 50nm;
the film catalyst, wherein the powdery catalyst contained in the catalyst layer is a catalytic active substance carried on a porous material;
the film catalyst, wherein the catalyst layer has a thickness of not more than 100 μm;
the film catalyst having a honeycomb structure;
the film catalyst, which is used as a catalyst for a gas-liquid reaction.

The second invention includes the following preferred embodiments:

the method for production, wherein the catalyst layer of the film catalyst has a pore size distribution with a mode value $t_2$ differing from the $t_1$ as determined by mercury porosimetry and satisfying the formula:

$$t_2 \leq 0.2 \times t_1;$$

the method for production, wherein the mode value $t_2$ is 4 to 50 nm;
the method for production, wherein the coating composition is prepared with a media-type mill;
the method for production, wherein the coating composition is prepared under the conditions satisfying the formula:

$$500 < \text{time} \cdot\cdot V \cdot P/G < 5000 \quad (\text{m} \cdot \text{L/kg})$$

wherein, time (sec.) is a period of preparation in the media-type mill, V (m/sec.) is a typical velocity, P (L) is an apparent volume of a media used in preparation and G (kg) is a blended mass of the coating composition;
the method for production further containing drying and/or curing;
the method for production further containing forming in shape and heating after the drying and/or curing;
the method for production, wherein the forming step is to form into a honeycomb structure;
the method for production, wherein the powdery catalyst contained in the coating composition is a catalytic active substance carried on a porous material;
the method for production, wherein the binder is contained in the coating composition in an amount of 10 to 40 % by mass;
the method for production, wherein a thickness of the catalyst layer of the film catalyst is not more than 100 μm;
the method for production, wherein the film catalyst is used as a catalyst for a gas-liquid reaction.

As used herein, the term "powdery catalyst" refers a powdery substance having a catalytic activity. The term "film catalyst" is not intended to limit a shape of the substrate to film, but means that a formed catalyst layer has a thin thickness.
The film catalyst of the first invention has a sufficient reactivity, a small amount of side-products are produced and a small amount of the catalyst falls off from the catalyst layer, since a detaching strength of the catalyst layer from the substrate is large.
A film catalyst obtained by the method for production of the second invention has a sufficient reactivity; a small amount of side-products is produced and a small amount of the catalyst falls off from the catalyst layer since a detaching strength of the catalyst layer from the substrate is large.

Brief Description of the Drawings

Fig. 1 shows a schematic cross-section view of the film catalyst obtained by the method for production of the present invention.

Fig. 2 shows a partial cross-section view of Fig. 1.

Fig. 3 shows a partial cross-section view of Fig. 2.

Fig. 4-1 shows a graph of a pore size distribution for illustrating the formula (1-I).

Fig. 4-2 shows a graph of a pore size distribution for illustrating the formula (2-I).

Fig. 5 shows an explanatory drawing for determining a mode value in the formulae (1-I) and (2-I).

Fig. 6 shows a perspective view of an embodiment of the film catalyst obtained by the method for production of the present invention.

Fig. 7 shows a perspective view of another embodiment of the film catalyst obtained by the method for production of the present invention.

Fig. 8 shows a perspective view of still another embodiment of the film catalyst obtained by the method for production of the present invention.

Fig. 9 shows a perspective view of a cylindrical reactor used in Examples 1-5 and 2-5, and Comparative Examples 1-5 and 2-5.

The present invention will be described below based on preferred embodiments with reference to Figures. In the following description, first, the film catalyst of the first invention is described, and second, the method for production of film catalyst of the second invention is described.

Detailed Description of the Invention

<The first invention: film catalyst>

A film catalyst of the first invention has a catalyst layer containing a powdery catalyst and a binder on one surface or both surfaces of a substrate.

Percentages of the powdery catalyst and the binder in the catalyst layer are substantially equated to those in the coating composition used in preparation. In a sum of both ingredients, the powdery catalyst preferably accounts for 60 to 90 % by mass, more preferably 60 to 85 % by mass, and even more preferably 70 to 85 % by mass, and the binder preferably accounts for 10 to 40 % by mass, more preferably 15 to 40 % by mass, and even more preferably 15 to 30 % by mass.

In the film catalyst of the first invention, the catalyst layer has a pore size distribution with two or more different mode values as determined by mercury porosimetry (a detailed description of the measurement method is provided in Examples).

As used herein, the term "mode value" refers a diameter at a peak value in a pore size distribution (e.g. , mode values $t_1$ and $t_2$ of peak values $Pt_1$ and $Pt_2$, respectively, in Fig. 4-1). In a pore size distribution, respective peak values are determined without data clearly considered as noises of a measuring devise (such as between 0.03 to 0.05 μm or 0.6 μm or more in pore size, in Fig. 4-1).

In the film catalyst of the first invention, the catalyst layer preferably has two or more different mode values as determined by mercury porosimetry, of which the maxim mode value $t_1$ and a mode value $t_2$ differing from the mode value $t_1$ satisfy the relationship represented by the formula (1-I):

$$t_2 \leq 0.2 \times t_1 \quad (1\text{-I}).$$

The catalyst layer of the film catalyst of the first invention satisfies the formula (1-I), because it has a layer structure. The relationship between the layer structure of the catalyst in the film catalyst of the first invention and the formula (1-I) will be described below with reference to Figures.

Fig. 1 is a schematic view of the film catalyst of the present invention (the first invention) cut across in the thickness direction, and shows the layer structure of the catalyst layer of the film catalyst. Fig. 2 shows an enlarged view of a part enclosed in a rectangular head in Fig. 1. Fig. 3 shows an enlarged view of a part enclosed in a rectangular head in Fig. 2.

Fig. 4-1 shows a pore size distribution with two or more different mode values as determined by mercury porosimetry. In Fig. 4-1, $t_1$ is the maximum mode value and $t_2$ is a mode value different from the mode value $t_1$, in the pore size distribution. Fig. 5 is a drawing for illustrating a method of determining a mode value.

As shown in Fig. 1, a film catalyst 1 has catalyst layers 3 on both sides of a substrate 2. The catalyst layer 3 may be on one side of the substrate 2. As shown in Fig. 2, the catalyst layer 3 contains a powdery catalyst 4 and a binder 5 surrounding particles of the powdery catalyst 4. In this figure, voids 6 shown as a space enclosed with a dashed line present among particles of the powdery catalyst 4 (and the binder 5 surrounding particles of the powdery catalyst 4). As shown in Fig. 3, the binder 5 surrounding particles of the powdery catalyst 4 has openings 7 shown as a space enclosed with a dashed line. At the part of the opening 7, the powdery catalyst 4 is not covered with the binder 5 and the surface thereof is exposed.

It is thought that $t_1$ (maxim mode value in the pore size distribution) shown in Fig. 4-1 is a peak derived from a void structure (void 6) presenting among particles of the powdery catalyst in the catalyst layer, and $t_2$ (mode value differing from $t_1$) is a peak derived from an opening structure (opening 7) generated by partially missing resin (binder) on the surface of the powdery catalyst.

In the film catalyst of the first invention, since the catalyst layer has the layer structure as shown in Figs. 1 to 3 (preferably, it satisfies the formula (1-I)), when the film catalyst is used as a catalyst for certain reaction, the void structure existing among powdery catalyst particles can surely provide mobility of a reactant to the deepest part of the catalyst layer.

Further, since the powdery catalyst has the opening structure generated by partially missing resin (binder) on the surface thereof, the surface has an exposed part to dramatically increase probability of contacting with the reactant migrating, resulting in enhanced progress of the chemical reaction at the surface. The opening structure generated by partial missing resin (binder) also allows a reaction product to smoothly move from the exposed surface of the catalyst to the void structure among powdery catalyst particles and further to the outside of the catalyst layer. The opening structure thus can dramatically increase mobility of the reactants and the reaction product.

In addition, easy and smooth moving of substances can provide prevention of overreaction of the reaction product in the catalyst layer to prevent generation of side-product, resulting in improved selectivity. In other words, it has been found in the firs invention that the layer structure has a feature that can achieve high reactivity and high reaction selectivity simultaneously.

As described above, probability of contact of the powdery catalyst in the catalyst layer of the film catalyst with the reactant is very high. This results in achievement of high reactivity and high selectivity, as demonstrated in Examples.

The film catalyst of the first invention can achieve higher reactivity preferably with a value of $t_2/ t_1$ not more than 0.2, more preferably not more than 0.15, and even more preferably not more than 0.1, when the relationship represented by the formula (1-I) is converted to $t_2/ t_1$.

From the viewpoint of achievement of high reactivity, the film catalyst of the first invention preferably has a mode value $t_1$ of 20 nm to 250,000 nm (250 $\mu$m), more preferably 100 nm to 100,000 nm (100 $\mu$m), and even more preferably 200 nm to 50,000 nm (50 $\mu$m). From the same viewpoint, the film catalyst of the first invention preferably has a mode value $t_2$ of 4 to 50 nm, and more preferably 4 to 30 nm.

The lower limit of $t_2/ t_1$ is, considering that a preferred upper limit of $t_1$ is 250 $\mu$m and a preferred lower limit of $t_2$ 4nm, $1.6\times10^{-5}$.

As shown in Fig. 5, for a mode value of an unplaceable peak, lines tangential to a plot are drawn, and the intersection of the two lines is considered as the mode value.

From the viewpoints of achievement of high reactivity and reduced amount of side-products, in the film catalyst of the first invention, the catalyst layer preferably has a thickness of not more than 500 $\mu$m, more preferably not more than 100 $\mu$m, and even more preferably not more than 50 $\mu$m. From the same viewpoints, an amount of catalyst carried per unit area is preferably 0.5 to 100 g/m$^2$, more preferably 1 to 60 g/m$^2$, and even more preferably 10 to 30 g/m$^2$.

The film catalyst of the first invention can be of any structure, including a laminate structure containing planar film catalysts and corrugated film catalysts prepared from the planar film catalysts that are alternatively layered several times (see Fig. 6), a cylindrical structure used for a flow reactor containing a catalyst layer and a cylinder on the inner surface of which the catalyst layer is formed (see Fig. 8), a sheet structure for separating the inner space of a cylinder into axial direction flow passages containing a catalyst layer and a sheet on which the catalyst layer is formed and a fin plate structure used in an open type reaction tank as placed in the tank containing a catalyst layer and a plate on which the catalyst layer is formed.

In any case, the structure may be partially constructed with a member not having a catalyst layer. The structure preferably allows easy supply of a reactant to and easy recovery of a product from the catalyst layer. It is desirable for efficient progress of reaction to increase a surface area of the catalyst layer of the film catalyst installed in a reactor as large as possible. A reactor desirably has a space serving as a flow passage for easy migration of a reactant and a product. To keep the flow passage and to secure strength of the whole catalyst, it is also preferable that film catalysts are adhered each other.

To satisfy the requirements, those structures may be used such as an assembly of bundled tubes having an internal diameter of several mm to several tens mm and a honeycomb structure having a cell density of several cells to two hundreds cells per a square centimeter, containing a catalyst layer formed on the inner wall of a tube and a cell.

The film catalyst of the first invention is applicable to a variety of reactions by selecting a powdery catalyst contained in the film catalyst. A powdery catalyst used for a reaction at a liquid phase is particularly preferably selected, because the resultant film catalyst can be used as is obtained without impairing diffusion properties of a reactant and a product, and can adapt to a thermal environment in the reactor. The film catalyst of the first invention is particularly suitable for a catalyst for gas-liquid reaction.

Examples of the reaction that the film catalyst of the first invention can be used in reaction including oxidation of olefins, oxidation of alcohols, isomerization of olefins, isomerization of aromatics, carbonylation, and hydrogenation of esters, and preferably include dehydrogenation and hydrogenation. The film catalyst of the first invention can also be used in amination to produce tertiary amines from primary and secondary amines with alcohols, containing dehydrogenation and hydrogenation.

<Method for producing film catalyst>

A method for producing the film catalyst of the first invention contains:

preparing a coating composition containing a powdery catalyst, a binder and a solvent;
applying the coating composition on a substrate and forming a film thereof to give a catalyst intermediate; and
drying (aging) at an ambient temperature; or
drying and curing; structure-forming; and heat-finishing, according to need. The film catalyst of the first invention can be produced by being subjected to these steps, for example, in this order and treated at each step. These steps can be optionally changed in order or skipped. These steps will be described individually below.

(Preparation of coating composition)

The coating composition used in production of the film catalyst of the first invention can be prepared as follows.

As the powdery catalyst contained in the coating composition, a catalytic active substance and a catalytic active substance carried on a porous material can be used.

The catalytic active substance can be any component as long as it is active on a chemical reaction to be applied. Examples of the component include metal elements such as Ag, Au, Cu, Ni, Fe, Al, transition metal elements of the Period 4, elements of the platinum group, elements of the Group IIIA, alkaline metals and alkaline earth metals in periodic table, and metal oxides thereof.

The porous material serves for a catalyst carrier that carries the catalytic active substance. Examples of the porous material include activated charcoal, alumina, silica, zeolite, titania, silica-alumina and diatomaceous earth. These may be preferably used alone or in combination. The porous material having high surface area is more preferably used. Examples also include molecular sieves.

Examples of a method of making the catalyst carrier carrying the catalytic active substance include known methods commonly used such as impregnation, co-impregnation, coprecipitation and ion-exchange.

From the viewpoints of achievement of high reactivity and reduced amount of side-products, the powdery catalyst preferably has an average particle diameter of 0.01 to 500 $\mu$m, more preferably 0.5 to 150 $\mu$m, and even more preferably 1 to 50 $\mu$m, with a sharp peak of distribution. From the same viewpoints, the powdery catalyst preferably has a relative surface area of 1 to 500 $m^2$/g, more preferably 5 to 200 $m^2$/g, and even more preferably 10 to 100 $m^2$/g, as determined by the BET method.

From the viewpoints of achievement of high reactivity and reduced amount of side-products, a percentage of the powdery catalyst contained in the coating composition is preferably 60 to 90 % by mass, more preferably 60 to 85 % by mass, and even more preferably 70 to 85 % by mass.

The binder used in the coating composition preferably has good binding properties as binding powdery catalyst particles each other and to the surface of the substrate, withstands reaction environments, and has no adverse effect on a reaction system. Examples of the binder include cellulose resins such as carboxymethylcellulose and hydroxyethyl cellulose; fluorinated resins such as poly(tetrafluoroethylene) and poly(vinylidene fluoride); various thermoplastic and thermosetting resins such as polyvinyl alcohols, urethane resins, epoxy resins, polyamide resins, polyimide resins, polyamideimide resins, polyester resins, phenol resins, melamine resins and silicone resins. Examples also include those that can be highly polymerized by cross-linking these synthetic resins with a curing agent. Among them, preferred are thermosetting resins such as phenol, furan and epoxy resins, and more preferred are thermosetting resins that cause condensation reaction in curing.

Use of the thermosetting resin as the binder results in an enhanced strength and binding properties of the coating due to an increased cross-linking density by curing, and when the thermosetting resin causes a condensation reaction in curing, effectively ensures a catalytic activity of the powdery catalyst due to the catalyst coating turns into porous by the condensation.

When the film catalyst obtained by the method for production described in the first invention is used in a reaction of an alcohol with a primary or secondary amine to produce a tertiary amine, examples of a preferred combination of the powdery catalyst and the binder include a combination of a ternary powdery catalyst of copper-nickel-ruthenium and a phenol resin.

A percentage of the binder contained in the coating composition is preferably 10 to 40 % by mass, more preferably 15 to 40 % by mass, and even more preferably 15 to 30 % by mass.

By controlling content percentages of the powdery catalyst and the binder within the range described above, exposure of the powdery catalyst can be controlled, an original catalyst activity power of the powdery catalyst can be ensured, and an effect of preventing dropout of the catalyst layer can be improved. More specifically, as shown in Figs. 1 to 3, the powdery catalyst can be controlled to have a covered part with the binder and an exposed part without the binder in the surface thereof, and the effects can be achieved.

When the content percentage of the binder is not more than 40 % by mass, the binder covers the surface of the powdery catalyst with an appropriate thickness or with an appropriate coating state of the binder as shown in Fig. 3, and a catalytic activity of the powdery catalyst can be sufficiently achieved to high level. When the content percentage of the binder is not less than 10 % by mass, the catalytic activity is sufficiently achieved, binding power among powdery catalyst particles or between the powdery catalyst and the substrate is increased, and amounts of a detached or partly fallen catalyst layer during a process of producing the film catalyst and during running a reaction is controlled.

The coating composition contains the solvent in addition to the powdery catalyst and the binder to improve a wetting property of the surface of the powdery catalyst, to dissolve the binder, and to facilitate uniform mixing of these ingredients.

The solvent can be any solvent as long as it does not have an adverse effect on the catalytic activity of the powdery catalyst. The solvent is selected from various water-miscible and water-immiscible solvents according to a type of the binder used. A film catalyst can be controlled by choice of the solvent. The solvent preferably well dissolves the binder. The solvent may be used in combination with other solvent. A catalyst layer can be controlled by appropriately choosing the solvent for the binder used.

Examples of the solvent include: water; alcohols such as methyl alcohol, ethyl alcohol, isopropyl alcohol, butyl alcohol and allyl alcohol; ketones such as acetone, methylethylketone (MEK) and methylisobutylketone (MIBK); glycols and derivatives thereof such as ethylene glycol, propylene glycol, diethylene glycol, polyethylene glycol, polypropylene glycol, diethylene glycol monoethyl ether, polypropylene glycol monoethyl ether, polyethylene glycol monoallyl ether and polypropylene glycol monoallyl ether; glycerols and derivatives thereof such as glycerol, glycerol monoethyl ether and glycerol monoallyl ether; ethers such as tetrahydrofuran and dioxane; hydrocarbons such as liquid paraffin, decane, decene, methylnaphthalene, decalin, kerosene, diphenylmethane, toluene, dimethylbenzene, ethylbenzene, diethylbenzene, propylbenzene, cyclohexane and partially hydrogenated triphenyl; silicone oils such as polydimethylsiloxane, partially octyl-substituted polydimethylsiloxane, partially phenyl-substituted polydimethylsiloxane and fluorosilicone oil; halogenated hydrocarbons such as chlorobenzene, dichlorobenzene, bromobenzene, chlorodiphenyl and chlorodiphenylmethane; fluorinated solvents such as DAILROL (DAIKIN INDUSTRIES, Ltd.) and DEMNUM (DAIKIN INDUSTRIES, Ltd.); ester compounds such as ethyl benzoate, octyl benzoate, dioctyl phthalate, trioctyl trimellitate, dibutyl sebacate, ethyl (meth)acrylate, butyl (meth) acrylate and dodecyl (meth) acrylate; and others such as dimethylformamide, N-methyl-pyrrolidone, acetonitrile and ethyl acetate.

The coating composition may further contain dispersion assistants such as a surfactant and a coupling agent, aggregates such as an inorganic particle and a fibrous material and pore-forming assistants such as a solvent having high boiling point. The coupling agent has an effect of improving physical properties by producing intramolecular cross-linking at an interface between an inorganic filler and an organic polymer matrix.

As the coupling agent, coupling agents well known can be used, including silane, titanate and aluminate coupling agents. These coupling agents may be used in combination, and may be diluted with a compatible organic solvent for concentration adjustment to be used.

The fibrous material used may be an organic or inorganic fiber. Examples of the organic fiber include polyamide fibers such as nylon 6 and nylon 66, aramid fibers, polyvinyl alcohol fibers, polyester fibers such as polyethylene terephthalate and polybutylene terephthalate fibers, polyarylate fibers, and polyethylene and polypropylene fibers such as poly (vinylidene chloride)-based, poly(vinyl chloride)-based, polyacrylonitrile-based and polyolefin-based. Examples of the organic fiber also include organic regenerated fibers such as cellulose-based rayon and acetate. Examples of the inorganic fiber include glass fibers, carbon fibers, active carbon fibers and ceramic fibers. The fiber is blended to exhibit an aggregate effect, resulting in an improved mechanical strength (coating strength) of the catalyst layer.

Preparation of the coating composition can be conducted by any method without limitation as long as the method can uniformly mix ingredients. In the first invention, the coating composition is preferably prepared with a media-type mill, a paint shaker, or the like. A media-type mill has a structure and a mechanism allowing uniform treatment of ingredients constituting the coating composition described above, and is suitable for controlling a particle diameter and a dispersing state of the powdery catalyst to control a pore size distribution of the catalyst layer of the film catalyst.

Examples of the media-type mill include a ball mill, an attriter, an AD mill, a twin AD mill, a basket mill, a twin basket mill, a handy mill, a grain mill, a Dyno mill, an Apex mill, a star mill, a Visco mill, a sand grinder, and a paint shaker using beads. In the first invention, dispersion machines other than the media-type mill may also be used, including a dissolver-type dispersion machine.

The coating composition is preferably prepared under the conditions satisfying the formula (1-II):

$$500 < \mathrm{time} \cdot V \cdot P/G < 5000\ (\mathrm{m \cdot L/kg}) \quad (1\text{-}II)$$

wherein, time (sec.) is a period of preparation in the media-type mill, V (m/sec.) is a peripheral speed, P (L) is an apparent volume of a media used in preparation and G (kg) is a blend mass of the coating composition. In the formula (1-II), the typical velocity refers a peripheral speed of a stirring part for a rotary mill or a velocity obtained by dividing a movement stroke by a moving time for a mill working with a reciprocating mechanism such as a paint shaker.

Preparation of the coating composition so as to satisfy the formula (1-II) allows mode values in a pore size distribution of the catalyst layer to be similar to mode values in a pore size distribution of the powdery catalyst itself. The preparation condition represented by the formula (1-II) is preferably $700 < \mathrm{time} \cdot V \cdot P/G < 4000$, and more preferably $1000 < \mathrm{time} \cdot V \cdot P/G < 3000$.

A solid content of the coating composition, which effects on formation of a porous structure and controls it in elimination of a solvent from a formed catalyst layer, is preferably 10 to 80 % by mass, more preferably 20 to 70 % by mass, and even more preferably 25 to 65 % by mass. A viscosity of the coating composition is appropriately selected within the range of, for example, 5 to 10,000 mPas, preferably 20 to 5000 mPas, and more preferably 50 to 1000 mPas, according to a method of application.

(Preparation of catalyst intermediate)

A catalyst intermediate is prepared by applying the coating composition on a substrate to form a film. It is then dried (aged) at ambient temperature. Various methods that are conventionally known can be used for application, including blade coating, roll coating, knife coating, bar coating, spray coating, dip coating, spin coating, comma coating, kiss coating, gravure coating and die coating.

The substrate used in the first invention is appropriately selected according to an intended form of the film catalyst. Those can be used including planar, tubular, honeycomb-type and monolith-type substrates. A thickness of the substrate is preferably, but not limited to, not more than 1 mm.

The planar substrate is not specifically limited as long as it has appropriate workability and durability and not affects adversely on a reaction system in which the film catalyst of the first invention is used. Examples of the planar substrate include copper, stainless steel and aluminum foils. From the points of workability and corrosion resistance, preferably used are copper and stainless steel foils.

Examples of the honeycomb-type and the monolith-type substrates include, but not limited to, those containing cordierite, carbon composite, mullite, clay, magnesia, talc, zirconia, spinel, alumina, silica, ceria, titania, tungsten, chromium, stainless steel, copper, aluminum and nickel.

As used herein, the honeycomb refers a structure made of many cells adjacent each other across a thin wall in a manner similar to the natural honey's nest. The honeycomb-type substrate can have large surface area per unit volume, and is preferred for the substrate used in the film catalyst. Equilateral triangle, equilateral pentagon and equilateral hexagon cells are preferably used, because they can be integrated with no space therebetween. The honeycomb-type substrate may also be constructed with a combination of cells having different shapes and various polygon cells. As the honeycomb-type substrate, those can be preferably used, for example, an integrated structure formed by extrusion molding and a substrate containing a planar material and a corrugated material (corrugate) prepared from the planar material alternatively layered several times.

From the viewpoint of increased adhesion of the substrate to the catalyst layer, a surface of the substrate is preferably subjected to a surface roughening treatment or coupling treatment. In the coupling treatment, coupling agents described above can be used. Preferably used is the same coupling agent to that used in preparing the coating composition.

The drying and curing is conducted after applying the coating composition on the substrate and forming a film to remove the solvent and unreacted monomers contained in the coating composition. In the drying, a curing reaction progresses in association with drying, or a curing reaction is allowed to partly progress simultaneously with drying by controlling a heating temperature and a heating time according to constitution of the coating composition.

The drying and curing is preferably conducted in an atmosphere of heated air, steam, or inert gas such as nitrogen or argon, or by blowing these heat media. Other methods may also be used, including methods of radiant heating with infrared and far-infrared radiations and of heating with an induced current by an electromagnetic wave. A combination of these methods may also be used. A method of naturally drying (air drying) at ambient temperature may also be used. Examples of an ingredient removed in the step include volatile ingredients, typically the solvent, and reaction products by the curing. Examples of the volatile ingredient other than the solvent include an unreacted monomer ingredient.

Conditions for the drying and curing must be regulated according to physical properties of the binder and volatile ingredients, typically the solvent, contained in the coating composition. A porous structure (volume) of the catalyst layer can be controlled according to selection of the solvent and setting of the drying and curing conditions. In a heat treatment with hot air, it is thought that the hot air having higher drying temperature and larger drying volume removes the above-mentioned ingredients from the catalyst layer faster, resulting in larger pores (larger pore diameter, larger pore volume), and the hot air having a lower drying temperature and a smaller drying volume results in smaller pores.

Pores are decided in progress of curing and cross-linking reaction to form a cross-linking structure (networking structure) and in removing a condensed product when condensation is also occurred, as well as in removing the volatile ingredients, typically the solvent, from the coating composition.

In the drying and curing treatment, a method and a condition of drying are selected that do not affect adversely on an original catalytic activity of the powdery catalyst used in the first invention.

In the first invention, conditions of drying and curing with hot air to give an intended film catalyst are typically at a temperature of 60 to 400˚C, preferably 70 to 250˚C, more preferably 80 to 150˚C, with a wind velocity of preferably 0.5 to 30 m/sec, more preferably 1 to 20 m/sec, preferably for a second or more, more preferably for 10 minutes or more, and even more preferably for 30 minutes or more.

When only drying is intended without curing, the coating composition is preferably dried immediately after applied on the substrate. A porous structure of a coating film can be controlled by regulating drying conditions in the step. A time from formation of the catalyst layer on the substrate to removal of the volatile ingredients, typically the solvent, is thus preferably shorter. The time is preferably within two hours, more preferably within 30 minutes, and even more preferably within 5 minutes.

In the first invention, when the binder contains a thermosetting resin, the catalyst intermediate is preferably processed into an intended shape at the state of containing uncured materials (state of prepolymer) in the catalyst layer prepared by applying and drying, and then subjected to a final heat treatment.

The drying and curing of the catalyst intermediate conducted before the final feat treatment may be stopped at the state of a part of the thermosetting resin is uncured. The thermosetting resin is preferably partly cured to improve retention and mechanical strength of the catalyst layer compared with that at the time of film formation until handling for shape forming can be performed. The volatile ingredients and the like may remain in the coating film at an order of several %.

Drying before the final heat treatment to give an intended film catalyst is typically conducted, for drying with hot air, under conditions at a temperature of 60 to 400˚C, preferably 70 to 250˚C, more preferably 80 to 150˚C, with a wind velocity of preferably 0.5 to 30 m/sec, more preferably 1 to 20 m/sec, preferably for 0.5 to 300 seconds, more preferably for 1 to 100 seconds.

Shape forming before complete curing of the catalyst layer of the film catalyst allows the catalyst layer to follow plastic deformation of the substrate, thereby improving shape processability. Further, complete curing after shape forming allows the catalyst layer to have a fixed cross-linking structure at the final stage of the process of production, thereby relieving residual stress in the catalyst layer to reduce an effect of the film catalyst on dropout of the catalyst layer.

In the first invention, when the binder contains a thermoplastic resin, the catalyst layer obtained by applying and drying is preferably processed into an intended shape, and then subjected to a final heat treatment. In the case of the catalyst layer containing the thermoplastic resin, the catalyst layer may have a residual stress generated according to plastic deformation of the substrate in shape forming, which the stress may affect adversely on retention of the catalyst layer. The final heat treatment can relieve the stress and make an entangled structure of the thermoplastic resin being stronger, thereby reducing an effect of the film catalyst on dropout of the catalyst layer.

The final heat treatment can be conducted after the catalyst intermediate is processed into a shape and variously assembled into configurations according to a form of reactor. For example, planar film catalysts and corrugated film catalysts prepared therefrom may be alternatively layered several times to form a laminate structure, which can be heat treated in a state of put in a holder or cassette for attaching to a reactor.

Conditions for the final heat treatment may be different according to a kind of the binder used. In the first invention, the final heat treatment is preferably conducted for 5 to 600 minutes, more preferably for 10 to 100 minutes, preferably at 80 to 400˚C, more preferably at 100 to 200˚C. Other methods such as radical polymerization, radiation polymerization and ultraviolet curing can be used instead of heat curing. In cases of using these methods, the catalyst layer can contain various polymerization catalysts according to need.

The film catalyst can be processed into various shapes according to forms of various reactors to be used for catalyst reactions. To obtain an intended shape, trimming, shaping, stacking and assembling can be used, according to need. The film catalyst can be processed into planar, tubular, honeycomb-type, and monolith-type structures. The film catalyst can also be put/filled in a holder or case to form a unit structure to be set in an actual reaction apparatus.
As described above, the first invention is illustrated in reference with preferred embodiments thereof, but not intended to be limited by the embodiments. For example, the film catalyst prepared by the method of the first invention can be used as a catalyst for liquid-liquid, gas-gas or solid-liquid reaction, in addition to a catalyst for gas-liquid reaction.
Next, the method for producing a film catslyst of the second invention will be described below based on preferred embodiments with reference to Figures.
In the following description, the method for producing a film catslyst of the second invention will be described for each step. The method may contain other treatment steps other than the steps described below within the scope that can solve a problem of the second invention.
In the second invention, the term "mode value" refers a diameter at a peak value in a pore size distribution (e.g. , mode values $T_1$, $t_1$ and $t_2$ of peak values P $T_1$, P $t_1$ and P $t_2$, respectively, in Fig.4-2). In a pore size distribution, respective peak values are determined without data clearly considered as noise of a measuring devise (e.g., peaks of diameters between 0.03 to 0.05 μm and of 0.6 μm or more, in Fig. 4-2).

<Step of preparing a coating composition>

In this step, the powdery catalyst, the binder, the solvent, and optional other ingredient as needed are uniformly mixed to prepare the coating composition.

[Powdery catalyst]

As the powdery catalyst, a catalytic active substance or a catalytic active substance carried on a porous material can be used.
The catalytic active substance can be any component as long as it is active on a chemical reaction to be applied. Examples of the component include metal elements such as Ag, Au, Cu, Ni, Fe, Al, transition metal elements of the Period 4, elements of the platinum group, elements of the Group IIIA in periodic table, alkaline metals and alkaline earth metals, and metal oxides thereof.
The porous material serves for a catalyst carrier that carries the catalytic active substance. Examples of the porous material include activated charcoal, alumina, silica, zeolite, titania, silica-alumina and diatomaceous earth. These may be preferably used alone or in combination. The porous material having high surface area is more preferably used. Examples also include molecular sieves.
Examples of a method of making the catalyst carrier carrying the catalytic active substance include known methods commonly used such as impregnation, co-impregnation, coprecipitation and ion-exchange.
From the viewpoints of achievement of high reactivity and reduced amount of side-products, the powdery catalyst preferably has an average particle diameter of 0.01 to 500 μm, more preferably 0.5 to 150 μm, and even more preferably 1 to 50 μm, with a sharp peak of distribution. From the same viewpoints, the powdery catalyst preferably has a relative surface area of 1 to 500 $m^2$/g, more preferably 5 to 200 $m^2$/g, and even more preferably 10 to 100 $m^2$/g, as determined by the BET method.
From the viewpoints of achievement of high reactivity and reduced amount of side-products, a percentage of the powdery catalyst contained in the coating composition is preferably 60 to 90 % by mass, more preferably 60 to 85 % by mass, and even more preferably 70 to 85 % by mass.

[Binder]

The binder preferably has good binding properties as binding powdery catalyst particles each other and to the surface of the substrate, withstands reaction environments, and has no adverse effect on a reaction system. Examples of the binder include cellulose resins such as carboxymethylcellulose and hydroxyethyl cellulose; fluorinated resins such as poly(tetrafluoroethylene) and poly(vinylidene fluoride); various thermoplastic and thermosetting resins such as polyvinyl alcohols, urethane resins, epoxy resins, polyamide resins, polyimide resins, polyamideimide resins, polyester resins, phenol resins, melamine resins and silicone resins. Examples also include those that can be highly polymerized by cross-linking these synthetic resins with a curing agent. Among them, preferred are thermosetting resins such as phenol, furan and epoxy resins, and more preferred are thermosetting resins that cause condensation reaction in curing.
Use of the thermosetting resin as the binder results in enhanced strength and binding properties of the coating due to increased cross-linking density by curing, and when the thermosetting resin causes condensation reaction in curing, effectively ensures a catalytic activity of the powdery catalyst due to the coating turns into porous by the condensation.

When the film catalyst obtained by the method for production of the second invention is used in a reaction of an alcohol with a primary or secondary amine to produce a tertiary amine, examples of a preferred combination of the powdery catalyst and the binder include a combination of a ternary powdery catalyst of copper-nickel-ruthenium and a phenol resin.

A percentage of the binder contained in the coating composition is preferably 10 to 40 % by mass, more preferably 15 to 40 % by mass, and even more preferably 15 to 30 % by mass.

By controlling content percentages of the powdery catalyst and the binder within the range described above, exposure of the powdery catalyst can be controlled, an original catalyst activity power of the powdery catalyst can be ensured, and an effect of preventing dropout of the catalyst layer can be improved. More specifically, as shown in Figs. 1 to 3, the powdery catalyst can be controlled to have a covered part with the binder and an exposed part without the binder in the surface thereof, and the effects such as development of catalyst activity power and dropout of the catalyst layer can be achieved.

When the content percentage of the binder is not more than 40 % by mass, the binder covers the surface of the powdery catalyst with an appropriate thickness or with an appropriate coating state as shown in Fig. 3, and a catalytic activity of the powdery catalyst can be sufficiently achieved to a high level. When the content percentage of the binder is not less than 10 % by mass, the catalytic activity is sufficiently achieved, binding power among powdery catalyst particles or between the powdery catalyst and the substrate is increased, and amounts of a detached or partly fallen catalyst layer during a process of producing the film catalyst and during running a reaction operation is controlled.

The coating composition contains the solvent in addition to the powdery catalyst and the binder to improve a wetting property of the surface of the powdery catalyst, to dissolve the binder, and to facilitate uniform mixing of these ingredients.

[Solvent]

The solvent can be any solvent as long as it does not have an adverse effect on the catalytic activity of the powdery catalyst. The solvent is selected from various water-miscible and water-immiscible solvents according to a type of the binder used. A film catalyst can be controlled by choice of the solvent. The solvent preferably well dissolves the binder. Two or more solvents may be used in combination. A catalyst layer can be controlled by choosing a solvent for the binder used.

Examples of the solvent include: water; alcohols such as methyl alcohol, ethyl alcohol, isopropyl alcohol, butyl alcohol and allyl alcohol; ketones such as acetone, methylethylketone (MEK) and methylisobutylketone (MIBK); glycols and derivatives thereof such as ethylene glycol, propylene glycol, diethylene glycol, polyethylene glycol, polypropylene glycol, diethylene glycol monoethyl ether, polypropylene glycol monoethyl ether, polyethylene glycol monoallyl ether and polypropylene glycol monoallyl ether; glycerols and derivatives thereof such as glycerol, glycerol monoethyl ether and glycerol monoallyl ether; ethers such as tetrahydrofuran and dioxane; hydrocarbons such as liquid paraffin, decane, decene, methylnaphthalene, decalin, kerosene, diphenylmethane, toluene, dimethylbenzene, ethylbenzene, diethylbenzene, propylbenzene, cyclohexane and partially hydrogenated triphenyl; silicone oils such as polydimethylsiloxane, partially octyl-substituted polydimethylsiloxane, partially phenyl-substituted polydimethylsiloxane and fluorosilicone oil; halogenated hydrocarbons such as chlorobenzene, dichlorobenzene, bromobenzene, chlorodiphenyl and chlorodiphenylmethane; fluorinated solvents such as DAILROL (DAIKIN INDUSTRIES, Ltd.) and DEMNUM (DAIKIN INDUSTRIES, Ltd.); ester compounds such as ethyl benzoate, octyl benzoate, dioctyl phthalate, trioctyl trimellitate, dibutyl sebacate, ethyl (meth)acrylate, butyl (meth) acrylate and dodecyl (meth) acrylate; and others such as dimethylformamide, N-methyl-pyrrolidone, acetonitrile and ethyl acetate.

The coating composition may further contain dispersion assistants such as a surfactant and a coupling agent, aggregates such as an inorganic particle and a fibrous material and pore-forming assistants such as a solvent having high boiling point. The coupling agent has an effect of improving physical properties by producing intramolecular cross-linking at an interface between an inorganic filler and an organic polymer matrix.

As the coupling agent, coupling agents well known can be used, including silane, titanate and aluminate coupling agents. These coupling agents may be used in combination, and may be diluted with a compatible organic solvent for concentration adjustment to be used.

The fibrous material used may be an organic or inorganic fiber. Examples of the organic fiber include polyamide fibers such as nylon 6 and nylon 66, aramid fibers, polyvinyl alcohol fibers, polyester fibers such as polyethylene terephthalate , polybutylene terephthalate fibers, polyarylate fibers, and polyethylene and polypropylene fibers such as poly(vinylidene chloride)-based, poly(vinyl chloride)-based, polyacrylonitrile-based and polyolefin-based. Examples of the organic fiber also includes organic regenerated fibers such as cellulose-based rayon and acetate. Examples of the inorganic fiber include glass fibers, carbon fibers, active carbon fibers and ceramic fibers. The fiber is blended to exhibit an aggregate effect, resulting in an improved mechanical strength (coating strength) of the catalyst layer.

[Method for preparation of coating composition]

The film catalyst obtained by the method for production of the second invention has the catalyst layer on the substrate at one side or both sides, and satisfies the production requirement of the formula (2-I) due to the layer structure of the catalyst layer:

$$0.55T_1< t_1<2T_1 \quad (2\text{-I})$$

wherein

$T_1$: the maximum mode value of the pore size distribution of the powdery catalyst itself used as a raw material contained in the catalyst layer of the film catalyst (as determined by mercury porosimetry) and

$t_1$: the maximum mode value of the pore size distribution of the catalyst layer of the film catalyst (as determined by mercury porosimetry).

The relationship between the layer structure of the film catalyst obtained by the method for production of the second invention and the formula (2-I) will be described below with reference to Figures.

Fig. 1 is a schematic view of the film catalyst obtained by the method for production according to the present invention (the second invention) cut across in the thickness direction, and shows the layer structure of the catalyst layer of the film catalyst. Fig. 2 shows an enlarged view of a part enclosed in a rectangular head in Fig. 1. Fig. 3 shows an enlarged view of a part enclosed in a rectangular head in Fig. 2.

Fig. 4-2 shows a pore size distribution as determined by mercury porosimetry (a detailed description of the measurement method is provided in Examples). In Fig. 4-2, $T_1$, $t_1$ and $t_2$ are mode values as described above. Fig. 5 is a drawing for illustrating a method of determining a mode value.

As shown in Fig. 1, a film catalyst 1 has catalyst layers 3 on both sides of a substrate 2. The catalyst layer 3 may be on one side of the substrate 2. As shown in Fig. 2, the catalyst layer 3 contains a powdery catalyst 4 and a binder 5 surrounding particles of the powdery catalyst 4. In this figure, voids 6 shown as a space enclosed with a dashed line present among particles of the powdery catalyst 4 (and the binder 5 surrounding particles of the powdery catalyst 4). As shown in Fig. 3, the binder 5 surrounding particles of the powdery catalyst 4 has openings 7 shown as a space enclosed with a dashed line. At the part of the opening 7, the powdery catalyst 4 is not covered with the binder 5 and the surface thereof is exposed.

It is thought that $t_1$ (maxim mode value in the pore size distribution of the catalyst layer of the film catalyst) shown in Fig. 4-2 is a peak derived from a void structure (void 6) presenting among particles of the powdery catalyst in the catalyst layer, and $t_2$ (mode value differing from $t_1$) is a peak derived from an opening structure (opening 7) generated by partially missing resin (binder) on the surface of the powdery catalyst.

In the film catalyst obtained by the method for production of the second invention, the catalyst layer has the layer structure as shown in Figs. 1 to 3. Further, $T_1$ (maxim mode value of the pore size distribution of the powdery catalyst itself used as a raw material contained in the catalyst layer of the film catalyst) has a similar value to $t_1$, or $T_1$ and $t_1$ can satisfy the formula (2-I). The similar value of $T_1$ to $t_1$ means that, when the film catalyst is used as a catalyst in certain reaction, the powdery catalyst present in the catalyst layer with keeping a particle shape of a raw material or of the powdery catalyst before preparation of the coating composition, and thus a void structure formed by particles of the catalyst and the binder in the catalyst layer is similar to a void structure of the powdery catalyst at a raw material state. In the film catalyst used as a catalyst for a reaction, the powdery catalyst thus can present as is and exhibit same performance as established at a raw material state.

In addition, the void structure existing among powdery catalyst particles can surely provide mobility of a reactant to the deepest part of the catalyst layer. Further, since the powdery catalyst has the opening structure generated by partially missing resin (binder) on the surface of the powdery catalyst, the surface of the powdery catalyst has an exposed part to dramatically increase probability of contacting with the reactant migrating at an active point, resulting in enhanced progress of the chemical reaction at the surface. The opening structure generated by partial missing resin (binder) also allows a reaction product to smoothly move from the exposed surface of the catalyst to the void structure among powdery catalyst particles and further to the outside of the catalyst layer. The opening structure thus can dramatically increase mobility of the reactant and the reaction product. Such easy and smooth moving of substances can provide prevention of overreaction of the reaction product in the catalyst layer to improve selectivity. In other words, the present inventors have found the method for producing the film catslyst having the layer structure that can keep an original reaction performance of the powdery catalyst as a raw material and highly extract the original reaction performance.

As described above, in the present invention, the powdery catalyst in the catalyst layer of the film catalyst has very high probability of contacting with the reactant, resulting in achievement of high reactivity and high selectivity, as clearly demonstrated in Examples.

The film catalyst obtained by the method for production of the second invention can achieve higher reactivity with the relationship represented by the formula (2-I) preferably satisfying $0.7T_1<t_1<2T_1$, and more preferably $0.8T_1<t_1<1.5T_1$.

From the viewpoint of achievement of high reactivity, the mode value $T_1$ is preferably 10 nm to 250,000 nm (250 μm), more preferably 200 nm to 50,000 nm (50 μm), and even more preferably 400 nm to 25,000 nm (25 μm).

From the viewpoint of achievement of high reactivity, the mode value $t_1$ is preferably 20 nm to 250,000 nm (250 μm), more preferably 100 nm to 100,000 nm (100 μm), and even more preferably 200 nm to 50,000 nm (50 μm).

In the film catalyst obtained by the method for production of the second invention, the catalyst layer of the film catalyst further has a mode value $t_2$ other than the maxim mode value $t_1$ in the pore size distribution as determined by mercury porosimetry. The mode value $t_2$ preferably satisfies $t_2 \leq 0.2 \times t_1$, and more preferably $t_2 \leq 0.15 \times t_1$. From the viewpoint of achievement of high reactivity, the mode value $t_2$ is preferably 4 to 50 nm, and more preferably 4 to 30 nm.

The lower limit of $t_2/ t_1$ is, considering that a preferred upper limit of $t_1$ is 250 μm and a preferred lower limit of $t_2$ is 4nm, $1.6 \times 10^{-5}$.

As shown in Fig. 5, for a mode value of an unplaceable peak, lines tangential to a plot are drawn, and the intersection of the two lines is considered as the mode value.

From the viewpoints of achievement of high reactivity and reduced amount of side-products, in the film catalyst obtained by the method for production of the second invention, the catalyst layer preferably has a thickness of not more than 500 μm, more preferably not more than 100 μm, and even more preferably not more than 50 μm. From the same viewpoints, an amount of catalyst carried per unit area is preferably 0.5 to 100 $g/m^2$, more preferably 1 to 60 $g/m^2$, and even more preferably 10 to 30 $g/m^2$.

Preparation of the coating composition can be conducted by any method without limitation as long as the method can uniformly mix ingredients. In the second invention, the coating composition is preferably prepared with a media-type mill, a paint shaker, or the like. A media-type mill has a structure and a mechanism allowing uniform treatment of ingredients constituting the coating composition described above, and is suitable for controlling a particle diameter and a dispersing state of the powdery catalyst to control a pore size distribution of the catalyst layer of the film catalyst.

Examples of the media-type mill include a ball mill, an attriter, an AD mill, a twin AD mill, a basket mill, a twin basket mill, a handy mill, a glain mill, a Dyno mill, an Apex mill, a star mill, a Visco mill, a sand grinder, and a paint shaker using beads. In the second invention, dispersion machines other than the media-type mill may also be used, including a dissolver-type dispersion machine.

The coating composition is preferably prepared under the conditions satisfying the formula (2-II):

$$500 < \text{time} \cdot V \cdot P/G < 5000 \ (\text{m} \cdot \text{L/kg}) \quad (2\text{-II})$$

wherein, time (sec.) is a period of preparation in the media-type mill, V (m/sec.) is a typical velocity, P (L) is an apparent volume of a media used in preparation and G (kg) is a blend mass of the coating composition.

In the formula (2-II), a typical velocity refers a peripheral speed of a stirring part for a rotary mill and a velocity obtained by dividing a movement stroke by a moving time for a mill working with a reciprocating mechanism such as a paint shaker.

In general, conditions for preparing a coating composition are often represented by the product of a shear rate ($sec^{-1}$) applied on the coating composition and a period of preparation (sec). For a media-type mill, determination of a shear rate and modeling of shear impression state are difficult. The present inventers have found that conditions for preparing a coating composition are effectively determined according to the formula (2-II) for a media-type mill. In the formula (2-II), conditions for preparing a coating composition are represented as the product of a typical velocity (V) of a stirring part, a period of preparation (time) and a ratio of an apparent volume of the media per a unit mass of the coating composition (P/G), which are applied on per a unit mass of the coating composition. The ratio of an apparent volume of the media per a unit mass of the coating composition (P/G) refers an amount of apparent volume of the media involved in preparation of the coating composition. Small value of apparent volume of the media for dispersing the coating composition relative to a weight of the coating composition (small P/G) means need of a sufficient typical velocity of the stirring part (V) and a sufficient period of preparation (time) to achieve good dispersion. In contrast, large value of apparent volume of the media (large P/G) allows small typical velocity of the stirring part (V) and/or short period of preparation (time). A dispersing effect of a media on the coating composition is influenced by a form, a density, a material or the like of the media used. The inventors of the second invention have focused attentions on an apparent volume of the media, which is the most dominant factor.

Just for a relationship between the typical velocity of the stirring part and the period of preparation, good conditions of coating composition are achieved for small value of typical velocity of the stirring part with long period of preparation.

The typical velocity (V) is preferably 0.1 to 20 m/sec, and more preferably 0.5 to 10 m/sec. The period of preparation (time) is 10 to 180 min, and preferably 10 to 90 min. The apparent volume P (L) of the media used for preparation and the blend mass G (kg) of the coating composition are not specifically limited, but a ratio of the apparent volume of the media to the coating composition per unit mass (P/G) is 1 to 9, preferably 2 to 8.

By preparing the coating composition so as to satisfy the formula (2-II), the maxim mode value $t_1$ (as determined by mercury porosimetry) in a pore size distribution of the catalyst layer of the film catalyst is still similar to the maxim mode value $T_1$ (as determined by mercury porosimetry) in a pore size distribution of the powdery catalyst itself used as a raw material contained in the catalyst layer of the film catalyst (see, Fig. 4-2). The preparation requirement represented by the formula (2-II) is preferably $700<\text{time}\cdot V\cdot P/G<4000$, and more preferably $1000<\text{time}\cdot V\cdot P/G<3000$.

A solid content of the coating composition, which effects on formation of pores and decides pores in elimination of a solvent from a formed catalyst layer, is preferably 10 to 80 % by mass, more preferably 20 to 70 % by mass, and even more preferably 25 to 65 % by mass. A viscosity of the coating composition is appropriately selected within the range of, for example, 5 to 10,000 mPas, more preferably 20 to 5000 mPas, and even more preferably 50 to 1000 mPas, according to a method of application.

<Step of forming a catalyst layer on a substrate to give a catalyst intermediate>

In the step, the above-described coating composition is used to form the catalyst layer on the substrate.

[Substrate]

The substrate used in the second invention is appropriately selected according to an intended form of the film catalyst. Those can be used including planar, tubular, honeycomb-type and monolith-type substrates. A thickness of the substrate is preferably, but not limited to, not more than 1 mm.

The planar substrate is not specifically limited as long as it has appropriate workability and durability and not affects adversely on a reaction system in which the film catalyst of the second invention is used. Examples of the planar substrate include copper, stainless steel and aluminum foils. From the points of workability and corrosion resistance, preferably used are copper and stainless steel foils.

Examples of the honeycomb-type and the monolith-type substrates include, but not limited to, those containing cordierite, carbon composite, mullite, clay, magnesia, talc, zirconia, spinel, alumina, silica, ceria, titania, tungsten, chromium, stainless steel, copper, aluminum and nickel.

As used herein, the honeycomb refers a structure made of many cells adjacent each other across a thin wall in a manner similar to the natural honey's nest. The honeycomb-type substrate can have large surface area per unit volume, and is preferred for the substrate used in the film catalyst. Equilateral triangle, equilateral pentagon and equilateral hexagon cells are preferably used, because they can be integrated with no space therebetween. The honeycomb-type substrate may also be constructed with a combination of cells having different shapes and various polygon cells. As the honeycomb-type substrate, those can be preferably used, for example, an integrated structure formed by extrusion molding or a substrate containing a planar material and a corrugated material (corrugate) prepared from the planar material alternatively layered several times.

From the viewpoint of increased adhesion of the substrate to the catalyst layer, a surface of the substrate is preferably subjected to a surface roughening treatment or coupling treatment. In the coupling treatment, coupling agents described above can be used. Preferably used is the same coupling agent to that used in preparing the coating composition.

[Method for preparation of catalyst layer]

As a method of forming a catalyst layer on the surface of the substrate, a method of applying the coating composition on the substrate to form a film can be used.

Various methods that are conventionally known can be used for application, including blade coating, roll coating, knife coating, bar coating, spray coating, dip coating, spin coating, comma coating, kiss coating, gravure coating and die coating.

<Drying and curing>

The method of the second invention may further contain drying and curing after applying the coating composition on the substrate surface to form a film, followed by shape-forming and final heating. In the case of drying (aging) at ambient temperature after applying and film-forming, the drying and curing is not required.

The drying and curing is conducted after applying the coating composition on the substrate and forming a film to remove the solvent and unreacted monomers contained in the coating composition. In the drying, a curing reaction progresses in association with drying, or a curing reaction is allowed to partly progress simultaneously with drying by controlling a heating temperature and a heating time according to constitution of the coating composition.

The drying and curing is preferably conducted in an atmosphere of heated air, steam, or inert gas such as nitrogen or argon, or by blowing these heat media. Other methods may also be used, including various methods of radiant heating with infrared and far-infrared radiations and of heating with an induced current by an electromagnetic wave. A combination of these methods may also be used. A method of naturally drying (air drying) at ambient temperature may also be used. Examples of an ingredient removed in the step include volatile ingredients, typically the solvent, and products by the curing reaction. Examples of the volatile ingredient other than the solvent include an unreacted monomer ingredient.

Conditions for the drying and curing must be regulated according to physical properties of the binder and volatile ingredients, typically the solvent, contained in the coating composition. A porous structure (volume) of the catalyst layer can be controlled according to selection of the solvent and setting of the drying and curing conditions. In a heat treatment with hot air, it is thought that the hot air having higher drying temperature and larger drying volume removes the above-mentioned ingredients from the catalyst layer faster, resulting in larger pores (larger pore diameter, larger pore volume), and the hot air having lower drying temperature and smaller drying volume results in smaller pores.

Pores are decided in progress of curing and cross-linking to form a cross-linking structure (networking structure) and in removing a condensed product when condensation is also occurred, as well as in removing the volatile ingredients, typically the solvent, from the coating composition.

In the drying and curing treatment, a method and a condition of drying are selected that does not affect adversely on an original catalytic activity of the powdery catalyst used in the second invention.

In the second invention, conditions of drying and curing with hot air to give an intended film catalyst are typically at a temperature of 60 to 400˚C, preferably 70 to 250˚C, more preferably 80 to 150˚C, with a wind velocity of preferably 0.5 to 30 m/sec, more preferably 1 to 20 m/sec, preferably for a second or more, more preferably for 10 minutes or more, and even more preferably for 30 minutes or more.

When only drying is intended without curing, the coating composition is preferably dried immediately after applied on the substrate. A porous structure of a formed film can be controlled by regulating drying conditions in the step. A time from formation of the catalyst layer on the substrate to removal of the volatile ingredients, typically the solvent, is thus preferably shorter. The time is preferably within two hours, more preferably within 30 minutes, and even more preferably within 5 minutes.

[Shape forming and final heating of catalyst intermediate]

In the second invention, when the binder contains a thermosetting resin, the catalyst intermediate is preferably processed into an intended shape at the state of containing uncured materials (state of prepolymer) in the catalyst layer prepared by applying and drying, and then subjected to a final heat treatment.

The drying and curing of the catalyst intermediate conducted before the final heat treatment may be stopped at the state of a part of the thermosetting resin is uncured. The thermosetting resin is preferably partly cured to improve retention and mechanical strength of the catalyst layer compared with that at the time of film formation until handling for shape forming can be performed. The volatile ingredients and the like may remain in the film at an order of several %.

Drying before the final heat treatment to give an intended film catalyst is typically conducted, for drying with hot air, under conditions at a temperature of 60 to 400˚C, preferably 70 to 250˚C, more preferably 80 to 150˚C, with a wind velocity of preferably 0.5 to 30 m/sec, more preferably 1 to 20 m/sec, preferably for 0.5 to 300 seconds, more preferably for 1 to 100 seconds.

Shape forming before complete curing of the catalyst layer of the film catalyst allows the catalyst layer to follow plastic deformation of the substrate, thereby improving shape processability. Further, complete curing after shape forming allows the catalyst layer to have a fixed cross-linking structure at the final stage of the process of production, thereby relieving residual stress in the catalyst layer to reduce an effect of the film catalyst on falling of the catalyst layer.

In the second invention, when the binder contains a thermoplastic resin, the catalyst layer obtained by applying and drying is preferably processed into an intended shape, and then subjected to a final heat treatment. In the case of the catalyst layer containing the thermoplastic resin, the catalyst layer can have a residual stress generated according to plastic deformation of the substrate in shape forming, which the stress may affect adversely on retention of the catalyst layer. The final heat treatment can relieve the stress and make an entangled structure of the thermoplastic resin being stronger, thereby reducing an effect of the film catalyst on dropout of the catalyst layer.

The final heat treatment can be conducted after the catalyst intermediate is processed into a shape and variously assembled into configurations according to a form of reactor. For example, planar film catalysts and corrugated film catalysts prepared therefrom are alternatively layered several times to form a laminate structure, which can be heat treated in a state of put in a holder or cassette for attaching to a reactor.

Conditions for the final heat treatment maybe different according to a kind of the binder used. In the second invention, the final heat treatment is preferably conducted for 5 to 600 minutes, more preferably for 10 to 100 minutes, preferably at 80 to 400°C, more preferably at 100 to 200°C. Other methods such as radical polymerization, radiation polymerization and ultraviolet curing can be used instead of heat curing. In cases of using these methods, the catalyst layer can contain various polymerization catalysts according to need.

The film catalyst can be processed into various shapes according to forms of various reactors to be used for catalyst reactions. To obtain an intended shape, trimming, shaping, stacking and assembling can be used, according to need. The film catalyst can be processed into planar, tubular, honeycomb-type, or monolith-type structures. The film catalyst can also be put/filled in a holder or case to form a unit structure to be set in an actual reaction apparatus.

The film catalyst prepared by the method for production of the second invention can be processed into any structure, including a laminate structure containing planar film catalysts and corrugated film catalysts prepared from the planar film catalysts that are alternatively layered several times (see Fig. 6), a cylindrical structure used for a flow reactor containing a catalyst layer and a cylinder on the inner wall of which the catalyst layer is formed (see Fig. 8), a sheet structure for separating the inner space of a cylinder into axial direction flow passages containing a catalyst layer and a sheet on which the catalyst layer is formed and a fin plate structure used in an open type reaction tank as placed in the tank containing a catalyst layer and a plate on which the catalyst layer is formed.

In any case, the structure preferably allows easy supply of a reactant to and easy recovery of a product from the catalyst layer. It is desirable for efficient progress of reaction to increase a surface area of the catalyst layer of the film catalyst installed in a reactor as large as possible. To satisfy the requirements, those structures may be used such as an assembly of bundled tubes each having an internal diameter of several mm to several tens mm and a honeycomb structure having a cell density of several cells to two hundreds cells per a square centimeter, containing a catalyst layer formed on the inner wall of a tube and a cell.

The film catalyst prepared by the method for production of the second invention is applicable to a variety of reactions by selecting a powdery catalyst contained in the film catalyst. A powdery catalyst used for a reaction at a liquid phase is particularly preferably selected, because the resultant film catalyst can be used as is obtained without impairing diffusion properties of a reactant and a product, and can adapt to a thermal environment in the reactor. This film catalyst prepared by the method of the second invention is particularly suitable for a catalyst for gas-liquid reaction.

Examples of the reaction that the film catalyst prepared by the method of the second invention can be used include oxidation of olefins, oxidation of alcohols, isomerization of olefins, isomerization of aromatics, carbonylation, and hydrogenation of esters, and preferably include dehydrogenation and hydrogenation. The film catalyst of the second invention can also be used in amination to produce tertiary amines from primary and secondary amines with alcohols, containing dehydrogenation and hydrogenation.

As described above, the second invention is illustrated in reference with preferred embodiments thereof, but not intended to be limited by the embodiments. For example, the film catalyst prepared by the method for production of the second invention can be used as a catalyst for liquid-liquid, gas-gas or solid-liquid reaction, in addition to a catalyst for gas-liquid reaction.

## Examples

The following Examples will illustrate embodiments of the present invention. Examples are described for illustrative purposes not for limitation of the present invention.

In the following description, Examples 1-1 to 1-5 and Comparative Examples 1-1 to 1-5 are first described for the film catalyst of the first invention, and next, Examples 2-1 to 2-5 and Comparative Examples 2-1 to 2-5 for the method for producing the film catalyst of the second invention.

Examples and Comparative Examples of the first invention

### Preparation Example 1-1 (preparation of powdery catalyst)

A powdery catalyst containing a ternary catalytic active substance of copper-nickel-ruthenium and a synthetic zeolite carrying them was prepared as follows.

To a 1L flask, which contained a synthetic zeolite (average particle diameter: 6 μm), was added an aqueous solution of copper nitrate, nickel nitrate and ruthenium chloride prepared such that a molar ratio of metal atoms was Cu : Ni: Ru = 4:1:0.01, and heated to rise a temperature with stirring. To this was added dropwise an aqueous solution of 10 % by mass sodium carbonate at 90°C with controlling pH of the mixture to 9 to 10. The mixture was aged for one hour and filtered to separate a precipitate. The precipitate was washed with water, dried for 10 hours at 80°C, and calcined for three hours at 600°C to give a powdery catalyst. The resultant powdery catalyst contained 50 % by mass of metal oxide and 50 % by mass of synthetic zeolite.

Preparation Examples 1-2 and 1-3 (preparation of powdery catalyst)

Powdery catalysts were similarly prepared as in Preparation Example 1-1, except that in Preparation Example 1-2, an average particle diameter of a synthetic zeolite was 3 μm, and in Preparation Example 1-3, an average particle diameter of a synthetic zeolite was 3 μm and a temperature at which an aqueous solution of 10 % by mass sodium carbonate was added was 80°C . The resultant powdery catalysts also contained a ternary catalytic active substance of copper-nickel-ruthenium and a synthetic zeolite carrying them.

Preparation Example 1-4 (preparation of phenol resin)

A resole-type phenol resin was prepared as follows. In a 4L flask, paraformaldehyde (F), phenol (P) and zinc acetate as a reaction catalyst (CAT) in a molar ratio of F/P/CAT=1.8/1.0/0.015 were stirred and reacted for 16 hours at 80°C to give a resole-type phenol resin.

Examples and Comparative Examples will be described bellow. Examples 1-1 to 1-4 and Comparative Examples 1-1 to 1-4 describe film catalysts that can be used in tertiary amine preparation, and Example 1-5 and Comparative Example 1-5 describe film catalysts that can be used in aldehyde preparation.

Examples 1-1

To a 250 ml wide-mouthed polyethylene bottle were added MIBK as a solvent, a phenol resin (PR-9480, SUMITOMO BAKELITE Co., Ltd.,) as a binder and the powdery catalyst prepared in Preparation Example 1-1 in this order. The phenol resin was added in such ratio as 20 parts by mass of involatile matter thereof to 80 parts by mass (65 g) of powdery catalyst. MIBK was added in such amount as a solid content in a mixture was 60 % by mass. Glass beads of 1 mm diameter (apparent volume: 65 ml) as a dispersing media were further added to the wide-mouthed polyethylene bottle.

The wide-mouthed polyethylene bottle was set in a paint shaker and subjected to a mixing and dispersing treatment for 30 minutes to give a coating composition.

The coating composition was applied on both sides of a copper foil (thickness: 40 μm, basis weight: 310 $g/m^2$), which was used as a substrate, with a bar coater, and treated for 30 seconds at 130°C in a hot air circulation-type thermostatic oven to give an intermediate for film catalyst.

The resultant intermediate for film catalyst (width: 65 mm × length: 160 mm) was processed into a corrugated sheet. The corrugated sheet and planar film catalyst intermediates having the same dimensions were laminated and rolled to form a cylindrical intermediate for film catalyst. 14 cylindrical intermediates each having an outer diameter 30 mm and a height 65 mm were prepared. These were subjected to a curing treatment for 90 minutes at 150°C in a dryer to produce film catalysts having a shape shown in Fig. 7.

In the resultant film catalyst, masses of the catalyst layer per unit area were 17.9 $g/m^2$ for single side and 35.8 $g/m^2$ for both sides. Amounts of the powdery catalyst carried per unit area were 14.3 $g/m^2$ for single side and 28.6 $g/m^2$ for both sides. In the 14 cylindrical film catalysts, an amount of the powdery catalyst carried was 8.2 g. An average thickness of the catalyst layer (single side) was 10 μm.

Example 1-2

To a 250 ml wide-mouthed polyethylene bottle were added MIBK as a solvent, a phenol resin (PR-9480, SUMITOMO BAKELITE Co., Ltd.,) as a binder and the powdery catalyst prepared in Preparation Example 1-1 in this order. The phenol resin was added in such ratio as 10 parts by mass of involatile matter thereof to 90 parts by mass (65 g) of powdery catalyst. MIBK was added in such amount as a solid content in a mixture was 60 % by mass. Glass beads of 1 mm diameter (apparent volume: 65 ml) as a dispersing media were further added to the wide-mouthed polyethylene bottle.

The wide-mouthed polyethylene bottle was set in a paint shaker and subjected to a mixing and dispersing treatment for 5 minutes to give a coating composition.

The coating composition was applied on a copper foil (thickness: 40 μm, basis weight: 310 $g/m^2$) , which was used as a substrate, with a bar coater, and treated for 30 seconds at 130°C in a hot air circulation-type thermostatic oven to form a catalyst layer, thereby giving an intermediate for film catalyst. Catalyst layers were formed on both sides of the copper foil by the same method.

The resultant intermediate for film catalyst (width: 65 mm × length: 160 mm) was processed into a corrugated sheet. The corrugated sheet and planar film catalyst intermediates having the same dimensions were laminated and rolled to form a cylindrical intermediate for film catalyst. 20 cylindrical intermediates each having an outer diameter 30 mm and a height 65 mm were prepared. These were subjected to a curing treatment for 90 minutes at 150°C in a dryer to produce film catalysts having a shape shown in Fig. 7.

In the resultant film catalyst, masses of the catalyst layer per unit area were 4.7 g/$m^2$ for single side and 9.4 g/$m^2$ for both sides. Amounts of the powdery catalyst carried per unit area were 4.2 g/$m^2$ for single side and 8.4 g/$m^2$ for both sides. In the 20 cylindrical film catalysts, an amount of the powdery catalyst carried was 3.5 g. An average thickness of the catalyst layer (single side) was 4 μm.

Example 1-3

To a 250 ml wide-mouthed polyethylene bottle were added MIBK as a solvent, the phenol resin as a binder prepared in Preparation Example 1-4 and the powdery catalyst prepared in Preparation Example 1-2 in this order. The phenol resin was added in such ratio as 32 parts by mass of involatile matter thereof to 68 parts by mass (65 g) of powdery catalyst. MIBK was added in such amount as a solid content in a mixture was 60 % by mass. Zirconia beads of 2 mm diameter (apparent volume: 65 ml) as a dispersing media were further added to the wide-mouthed polyethylene bottle. The wide-mouthed polyethylene bottle was set in a paint shaker and subjected to a mixing and dispersing treatment for 110 minutes to give a coating composition.

The coating composition was applied on a copper foil (thickness: 40 μm, basis weight: 310 g/$m^2$), which was used as a substrate, with a bar coater, and treated for 30 seconds at 130°C in a hot air circulation-type thermostatic oven to form a catalyst layer, thereby giving an intermediate for film catalyst. Catalyst layers were formed on both sides of the copper foil by the same method.

The resultant intermediate for film catalyst (width: 65 mm × length: 150 mm) was processed into a corrugated sheet. The corrugated sheet and planar film catalyst intermediates having the same dimensions were laminated and rolled to form a cylindrical intermediate for film catalyst. 12 cylindrical intermediates each having an outer diameter 30 mm and a height 65 mm were prepared. These were subjected to a curing treatment for 90 minutes at 150°C in a dryer to produce film catalysts having a shape shown in Fig. 7.

In the resultant film catalyst, masses of the catalyst layer per unit area were 9.6 g/$m^2$ for single side and 19.3 g/$m^2$ for both sides. Amounts of the powdery catalyst carried per unit area were 6.6 g/$m^2$ for single side and 13.2 g/$m^2$ for both sides. In the 12 cylindrical film catalysts, an amount of the powdery catalyst carried was 3.1 g. An average thickness of the catalyst layer (single side) was 4 μm.

Example 1-4

To a 250 ml wide-mouthed polyethylene bottle were added MIBK as a solvent, a phenol resin (PR-9480, SUMITOMO BAKELITE Co., Ltd.,) as a binder and the powdery catalyst prepared in Preparation Example 1-2 in this order. The phenol resin was added in such ratio as 32 parts by mass of involatile matter thereof to 68 parts by mass (65 g) of powdery catalyst. MIBK was added in such amount as a solid content in a mixture was 60 % by mass. Zirconia beads of 2 mm diameter (apparent volume: 65 ml) as a dispersing media were further added to the wide-mouthed polyethylene bottle.

The wide-mouthed polyethylene bottle was set in a paint shaker and subjected to a mixing and dispersing treatment for 110 minutes to give a coating composition.

The coating composition was applied on both sides of a copper foil (thickness: 40 μm, basis weight: 310 g/$m^2$), which was used as a substrate, with a bar coater, and treated for 30 seconds at 130°C in a hot air circulation-type thermostatic oven to give an intermediate for film catalyst.

The resultant intermediate for film catalyst (width: 65 mm × length: 150 mm) was processed into a corrugated sheet. The corrugated sheet and planar film catalyst intermediates having the same dimensions were laminated and rolled to form a cylindrical intermediate for film catalyst. 12 cylindrical intermediates each having an outer diameter 30 mm and a height 65 mm were prepared. These were subjected to a curing treatment for 90 minutes at 150°C in a dryer to give film catalysts having a shape shown in Fig. 7.

In the resultant film catalyst, masses of the catalyst layer per unit area were 12.0 g/$m^2$ for single side and 24.0 g/$m^2$ for both sides. Amounts of the powdery catalyst carried per unit area were 8.2 g/$m^2$ for single side and 16.4 g/$m^2$ for both sides. In the 12 cylindrical film catalysts, an amount of the powdery catalyst carried was 3.8 g. An average thickness of the catalyst layer (single side) was 6 μm.

Example 1-5

To a 250 ml wide-mouthed polyethylene bottle were added MIBK as a solvent, a phenol resin (PR-9480, SUMITOMO BAKELITE Co., Ltd.,) as a binder and the powdery catalyst prepared in Preparation Example 1-1 in this order. The phenol resin was added in such ratio as 20 parts by mass of involatile matter thereof to 80 parts by mass (65 g) of powdery catalyst. MIBK was added in such amount as a solid content in a mixture was 60 % by mass. Glass beads of 1 mm diameter (apparent volume: 65 ml) as a dispersing media were further added to the wide-mouthed polyethylene bottle.

The wide-mouthed polyethylene bottle was set in a paint shaker and subjected to a mixing and dispersing treatment for 30 minutes to give a coating composition.

The coating composition was applied on both sides of a copper foil (thickness : 40 μm, basis weight: 310 g/m$^2$), which was used as a substrate, with a bar coater, and treated for 30 seconds at 130˚C in a hot air circulation-type thermostatic oven to give an intermediate for film catalyst (the same intermediate as in Example 1-1).

The resultant intermediate for film catalyst (width: 130 mm × length: 840 mm) was processed into a planar and corrugated sheet. The corrugated and planar sheets were alternatively laminated and put in a stainless-steel cylindrical reactor having an inner diameter 21.4 mm and a length 390 mm (Fig. 9). The reactor was subjected to a curing treatment for 90 minutes at 150˚C in a hot air circulation-type thermostatic oven to give a film catalyst.

In the resultant film catalyst, masses of the catalyst layer per unit area were 17.9 g/m$^2$ for single side and 35.8 g/m$^2$ for both sides. Amounts of the powdery catalyst carried per unit area were 14.3 g/m$^2$ for single side and 28.6 g/m$^2$ for both sides. In the film catalyst put in the cylindrical reactor, a mass of the powdery catalyst in the film catalyst was 3.1 g. An average thickness of the catalyst layer (single side) was 10 μm.

Comparative Example 1-1

To a 250 ml wide-mouthed polyethylene bottle were added MIBK as a solvent, a phenol resin (PR-9480, SUMITOMO BAKELITE Co., Ltd.,) as a binder and the powdery catalyst prepared in Preparation Example 1-3 in this order. The phenol resin was added in such ratio as 35 parts by mass of involatile matter thereof to 65 parts by mass (65 g) of powdery catalyst. MIBK was added in such amount as a solid content in a mixture was 60 % by mass. Zirconia beads of 2 mm diameter (apparent volume: 65 ml) as a dispersing media were further added to the wide-mouthed polyethylene bottle.

The wide-mouthed polyethylene bottle was set in a paint shaker and subjected to a mixing and dispersing treatment for 110 minutes to give a coating composition.

The coating composition was applied on both sides of a copper foil (thickness: 40 μm, basis weight: 310 g/m$^2$), which was used as a substrate, with a bar coater, and treated for 30 seconds at 130˚C in a hot air circulation-type thermostatic oven to give an intermediate for film catalyst.

The resultant intermediate for film catalyst (width: 65 mm × length: 150 mm) was processed into a corrugated sheet. The corrugated sheet and planar film catalyst intermediates having the same dimensions were laminated and rolled to form a cylindrical intermediate for film catalyst. 13 cylindrical intermediates each having an outer diameter 30 mm and a height 65 mm were prepared. These were subjected to a curing treatment for 90 minutes at 150˚C in a dryer to give film catalysts having a shape shown in Fig. 7.

In the resultant film catalyst, masses of the catalyst layer per unit area were 11.2 g/m$^2$ for single side and 22.4 g/m$^2$ for both sides. Amounts of the powdery catalyst carried per unit area were 7.3 g/m$^2$ for single side and 14.6 g/m$^2$ for both sides. In the 13 cylindrical film catalysts, an amount of the powdery catalyst carried was 3.8 g. An average thickness of the catalyst layer (single side) was 4 μm.

Comparative Example 1-2

To a 250 ml wide-mouthed polyethylene bottle were added MIBK as a solvent, a phenol resin (PR-9480, SUMITOMO BAKELITE Co., Ltd.,) as a binder and the powdery catalyst prepared in Preparation Example 1-2 in this order. The phenol resin was added in such ratio as 32 parts by mass of involatile matter thereof to 68 parts by mass (65 g) of powdery catalyst. MIBK was added in such amount as a solid content in a mixture was 60 % by mass. Zirconia beads of 2 mm diameter (apparent volume: 65 ml) as a dispersing media were further added to the wide-mouthed polyethylene bottle.

The wide-mouthed polyethylene bottle was set in a paint shaker and subjected to a mixing and dispersing treatment for 180 minutes to give a coating composition.

The coating composition was applied on both sides of a copper foil (thickness: 40 μm, basis weight: 310 g/m$^2$), which was used as a substrate, with a bar coater, and treated for 30 seconds at 130˚C in a hot air circulation-type thermostatic oven to give an intermediate for film catalyst.

The resultant intermediate for film catalyst (width: 65 mm × length: 150 mm) was processed into a corrugated sheet. The corrugated sheet and planar film catalyst intermediates having the same dimensions were laminated and rolled to form a cylindrical intermediate for film catalyst. 12 cylindrical intermediates each having an outer diameter 30 mm and a height 65 mm were prepared. These were subjected to a curing treatment for 180 minutes at 210˚C in a dryer to give film catalysts having a shape shown in Fig. 7.

In the resultant film catalyst, masses of the catalyst layer per unit area were 12.9 g/m$^2$ for single side and 25.9

g/m$^2$ for both sides. Amounts of the powdery catalyst carried per unit area were 8.8 g/m$^2$ for single side and 17.6 g/m$^2$ for both sides. In the 12 cylindrical film catalysts, an amount of the powdery catalyst carried was 4.1 g. An average thickness of the catalyst layer (single side) was 6 $\mu$m.

Comparative Example 1-3

To a 250 ml wide-mouthed polyethylene bottle were added MIBK as a solvent, a phenol resin (PR-9480, SUMITOMO BAKELITE Co., Ltd.,) as a binder and the powdery catalyst prepared in Preparation Example 1-1 in this order. The phenol resin was added in such ratio as 5 parts by mass of involatile matter thereof to 95 parts by mass (65 g) of powdery catalyst. MIBK was added in such amount as a solid content in a mixture was 60 % by mass. Glass beads of 1 mm diameter (apparent volume: 65 ml) as a dispersing media were further added to the wide-mouthed polyethylene bottle.

The wide-mouthed polyethylene bottle was set in a paint shaker and subjected to a mixing and dispersing treatment for 5 minutes to give a coating composition. The resultant coating composition contained many particles (flocculants).

The coating composition was applied on both sides of a copper foil (thickness: 40 $\mu$m, basis weight: 310 g/m$^2$), which was used as a substrate, with a bar coater, and treated for 30 seconds at 130˚C in a hot air circulation-type thermostatic oven to give an intermediate for film catalyst.

The resultant intermediate for film catalyst (width: 65 mm $\times$ length: 150 mm) was processed into a corrugated sheet. The corrugated sheet and planar film catalyst intermediates having the same dimensions were laminated and rolled to form a cylindrical intermediate for film catalyst. 14 cylindrical intermediates each having an outer diameter 30 mm and a height 65 mm were prepared. These were subjected to a curing treatment for 180 minutes at 210˚C in a dryer to give film catalysts having a shape shown in Fig. 7.

In the resultant film catalyst, masses of the catalyst layer per unit area were 9.0 g/m$^2$ for single side and 18. 0 g/m$^2$ for both sides. Amounts of the powdery catalyst carried per unit area were 8.6 g/m$^2$ for single side and 17.2 g/m$^2$ for both sides. In the 14 cylindrical film catalysts, an amount of the powdery catalyst carried was 4.6 g. An average thickness of the catalyst layer (single side) was 8 $\mu$m. The catalyst layer of the resultant film catalyst was very fragile and had poor strength. Although the resultant film catalyst was subjected to the evaluation for reaction characteristics as described below, it could not be properly evaluated as a catalyst for fixed-bed reaction due to much dropout of the catalyst layer.

Comparative Example 1-4

To a 250 ml wide-mouthed polyethylene bottle were added MIBK as a solvent, a phenol resin (PR-9480, SUMITOMO BAKELITE Co., Ltd.,) as a binder and the powdery catalyst prepared in Preparation Example 1-3 in this order. The phenol resin was added in such ratio as 50 parts by mass of involatile matter thereof to 50 parts by mass (65 g) of powdery catalyst. MIBK was added in such amount as a solid content in a mixture was 50 % by mass. Zirconia beads of 2 mm diameter (apparent volume: 65 ml) as a dispersing media were further added to the wide-mouthed polyethylene bottle.

The wide-mouthed polyethylene bottle was set in a paint shaker and subjected to a mixing and dispersing treatment for 250 minutes to give a coating composition.

The coating composition was applied on both sides of a copper foil (thickness: 40 $\mu$m, basis weight: 310 g/m$^2$), which was used as a substrate, with a bar coater, and treated for 30 seconds at 130˚C in a hot air circulation-type thermostatic oven to give an intermediate for film catalyst.

The resultant intermediate for film catalyst (width: 65 mm $\times$ length: 150 mm) was processed into a corrugated sheet. The corrugated sheet and planar film catalyst intermediates having the same dimensions were laminated and rolled to form a cylindrical intermediate for film catalyst. 13 cylindrical intermediates each having an outer diameter 30 mm and a height 65 mm were prepared. These were subjected to a curing treatment for 90 minutes at 150˚C in a dryer to give film catalysts having a shape shown in Fig. 7.

In the resultant film catalyst, masses of the catalyst layer per unit area were 15.9 g/m$^2$ for single side and 31.6 g/m$^2$ for both sides. Amounts of the powdery catalyst carried per unit area were 7.9 g/m$^2$ for single side and 15.8 g/m$^2$ for both sides. In the 13 cylindrical film catalysts, an amount of the powdery catalyst carried was 4 g. An average thickness of the catalyst layer (single side) was 11 $\mu$m.

Comparative Example 1-5

To a 250 ml wide-mouthed polyethylene bottle were added MIBK as a solvent, a phenol resin (PR-9480, SUMITOMO BAKELITE Co., Ltd.,) as a binder and the powdery catalyst prepared in Preparation Example 1-2 in this order. The phenol resin was added in such ratio as 32 parts by mass of involatile matter thereof to 68 parts by mass (65 g) of powdery catalyst. MIBK was added in such amount as a solid content in a mixture was 60 % by mass. Zirconia beads of 2 mm diameter (apparent volume: 65 ml) as a dispersing media were further added to the wide-mouthed polyethylene bottle.

The wide-mouthed polyethylene bottle was set in a paint shaker and subjected to a mixing and dispersing treatment for 180 minutes to give a coating composition.

The coating composition was applied on both sides of a copper foil (thickness: 40 μm, basis weight: 310 $g/m^2$), which was used as a substrate, with a bar coater, and treated for 30 seconds at 130˚C in a hot air circulation-type thermostatic oven to give an intermediate for film catalyst (the same intermediate as in Comparative Example 1-2).

The resultant intermediate for film catalyst (130 mm × 840 mm) was processed into a planar and corrugated sheet. The corrugated and planar sheets were alternatively laminated and put in a stainless-steel cylindrical reactor having an inner diameter 21.4 mm and a length 390 mm (Fig. 9) . The reactor was subjected to a curing treatment for 90 minutes at 150˚C in a hot air circulation-type thermostatic oven to give a film catalyst.

In the resultant film catalyst, masses of the catalyst layer per unit area were 12.9 $g/m^2$ for single side and 25.9 $g/m^2$ for both sides. Amounts of the powdery catalyst carried per unit area were 8.8 $g/m^2$ for single side and 17.6 $g/m^2$ for both sides. In the 12 cylindrical film catalysts, an amount of the powdery catalyst carried was 1. 9g. An average thickness of the catalyst layer (single side) was 6 μm.

(Evaluation for reaction characteristics 1-1)

Cylindrical film catalysts prepared in Examples 1-1 to 1-4 and Comparative Examples 1-1 to 1-4 each have a vertical flow passages across the length of cylinder, which passage has a cross-section area of about 0.1 $cm^2$.

In a glass reactor having an inner diameter of 130 mm, the bottom surface of which was a glass filter (2G), the cylindrical film catalyst and 1000 g of dodecyl alcohol (KALCOL 2098, KAO CORPORATION) as a raw material were placed. The catalyst was reductively activated. To the reactor were supplied a hydrogen gas and dimethyl amine with keeping a steady state at 220˚C.

Samples were repeatedly collected from the reactor over time and analyzed by gas chromatography. A concentration change of dodecyl alcohol to a reaction time, a mass of dodecyl alcohol used in the reaction M=1000 [g], an amount of powdery catalyst in the film catalyst used in the reaction G (g) were used to calculate a reaction rate constant k [/Hr·($M^2$/kg-ROH)] by the calculation formula below.

$$K = \frac{-\ln(C2/C2)}{(\text{Time } 2\text{-Time } 1)\cdot(G/M\times 100)}\ [/\text{Hr}\cdot\%]$$

wherein, Time1 and Time2 (Hr) are sampling points of time [Time2>Time1]; and C1 and C2 (%) are concentrations of dodecyl alcohol at the points of Time1 and Time2, respectively.

The reaction was considered as had ended when a concentration of dodecyl alcohol reached to 1 %, and those were also determined: TIME (Hr), a reaction time from the reaction start to the reaction end; M2 (%), a concentration of side-product, dilaurylmonomethylamine, at the reaction end as determined by gas chromatography; and TIME × M2, [a reaction time × a concentration of side-product]. Results are shown in Table 1-1 below.

The index, TIME × M2, is introduced for evaluating the catalyst, because a catalyst resulting in a shorter reaction time and a smaller amount of side-product in the reaction is considered as having better reaction characteristics. A catalyst having a smaller value of the index exhibits better reaction characteristics. In Table 1-1, the total of content percentages of the powdery catalyst and the binder is 100 % by mass. A film strength is evaluated as a "detaching strength". × refers generation of detachment of a catalyst layer and a cloudy reaction mixture due to detaching of catalyst layer. O refers no visually recognizable detachment and an unclouded reaction mixture after evaluation of the reaction.

(Evaluation for reaction characteristics 1-2)

To the film catalyst prepared in Example 1-5 put in the cylindrical reactor was flowed dodecyl alcohol (KALCOL 2098, KAO CORPORATION) at a flow rate 73.9 g/Hr and a hydrogen gas at a flow rate 33.6 NL/Hr to reductively activate the catalyst.

Then, hydrogen gas supply was stopped. To the cylindrical reactor were supplied an $N_2$ gas at 9 NL/Hr and dodecyl alcohol at 73.9 g/Hr with increasing a temperature to 220˚C. The reaction system was hold at the temperature to continuously progress dehydrogenation. A reaction pressure is an ambient pressure.

A sample taken from the reaction mixture was analyzed by gas chromatography. A concentration of dodecyl alcohol was 58.4 %, a concentration of aldehyde produced was 16.1 %. Considering a concentration of dodecyl alcohol used as a raw material was 98.6 %, the followings were calculated using these values:

reaction rate (%) = concentration of dodecyl alcohol as a raw material - concentration of dodecyl alcohol after the reaction

=98.6-58.4=40.2 (%)

selectivity (%) = concentration of aldehyde after the reaction (%) / reaction rate (%)× 100

=16.1/40.2×100=39.9 (%).

Results are shown in Table 1-2 below.

In Table 1-2, the total of content percentages of the powdery catalyst and the binder is 100 % by mass. Detaching strength in the Table 1-2 refers the same meaning as in Table 1-1.

The index, reaction rate × selectivity, is introduced for evaluating the catalyst, because a catalyst resulting in a higher reaction rate and a higher selectivity in the reaction is considered as having better reaction characteristics. A catalyst having a larger value of the index exhibits better reaction characteristics. The reason of using a different index from the index in "Evaluation for reaction characteristics 1-1" is that the reaction system differs from 1-1 and cannot be evaluated by TIME × M2.

(Evaluation for reaction characteristics 1-3)

Similarly as in Evaluation for reaction characteristics 1-2, to the film catalyst prepared in Comparative Example 1-5 put in the cylindrical reactor was flowed dodecyl alcohol (KALCOL 2098, KAO CORPORATION) at a flow rate 73.9 g/Hr and a hydrogen gas at a flow rate 33.6 NL/Hr to reductively activate the catalyst.

Then, hydrogen gas supply was stopped. To the cylindrical reactor were supplied an $N_2$ gas at 9 NL/Hr and dodecyl alcohol at 73.9 g/Hr with increasing a temperature to 220˚C. The reaction system was hold at the temperature to continuously progress dehydrogenation. A reaction pressure is an ambient pressure.

A sample taken from the reaction mixture was analyzed by gas chromatography. A concentration of dodecyl alcohol was 95.3 %, and a concentration of aldehyde produced was 0.8 %. Considering a concentration of dodecyl alcohol used as a raw material was 98.6 %, the followings were calculated using these values:

reaction rate (%) = concentration of dodecyl alcohol as a raw material - concentration of dodecyl alcohol after the reaction

=98.6-95.3=3.3 (%)

$$\text{selectivity (\%)} = \text{concentration of aldehyde after the reaction (\%)} / \text{reaction rate (\%)} \times 100 = 0.8/3.3 \times 100 = 24.2\ (\%).$$

Results are shown in Table 1-2 below.

(Method for measuring a pore size distribution)

A pore size distribution was measured in accordance with the method (mercury porosimetry) described, for example, in "Busshitsu no kinousei (functionality of material), Jikken Kagaku Kouza (Experimental Chemistry) the 4th ed., ed by the Chemical Society of Japan, Maruzen, Tokyo, vol. 12, p.486". More specifically, a pore size distribution can be measured with a special measuring apparatus such as a mercury intrusion porosimeter (Poresizer9320) manufactured by Shimadsu Corporation. The film catalyst was previously measured for a basis weight thereof and a basis weight and an area of copper foil before application of the coating composition and measured for a mass of the catalyst layer. A pore size distribution of the catalyst layer was measured by mercury porosimetry. In mercury porosimetry, a pore size was calculated by the calculation formula below:

$$D = -4\gamma \cos\theta / P$$

wherein, D represents a pore size, $\gamma$ represents a mercury surface tension, $\theta$ represents a contact angle and P represents a pressure. In the measurement, a mercury surface tension was 482.536 dyn/cm, a contact angle used was 130°, and a mercury pressure was from 0 to 30000 psia.

Based on the principle described above, a pore size distribution is determined by putting pressure upon mercury and measuring a volume of mercury intruding pores, or a volume V, with gradually varying the pressure, plotting a pore size D calculated according to the calculation formula against a volume, finding a derivative dV/d(logD) of the plotted curve, and making a graph with a vertical axis of the derivative against a horizontal axis of the pore size D (see, Fig. 4-1). This method is mercury porosimetry. A pore size distribution was measured in the range from 6 nm to 10000 nm of pore size. In Figs. 4-1 and 5, a unit (ml) in the volume refers an amount of intruding mercury and (g) refers a mass of sample of the catalyst layer used in the measurement. "NO DETECT" means that a mode value cannot be visually recognized in the graph.

In pore size distributions having different mode values (peaks of the curve plotted in the graph) of the catalyst layer measured by the method, a mode value in a pore size distribution which value was maximum was set to a mode value $t_1$, and a mode value smaller than $t_1$ but having a largest peak among the other mode values was set to a mode value $t_2$.

Table 1-1

| | Powdery catalyst (%) | Amount of binder (%) | maximum mode value t1 of catalyst layer (nm) | mode value t2 of catalyst layer (nm) | $t_2/t_1$ | Reaction characteristics | | | | |
|---|---|---|---|---|---|---|---|---|---|---|
| | | | | | | Reaction activity k (/Hr·%) | reaction time TIME (Hr) | side-product M2 (%) | TIME × M2 | Detaching strength |
| Example1-1 | 80 | 20 | 477 | 11 | 0.023 | 3.8 | 2.4 | 8.1 | 19.4 | ○ |
| Example1-2 | 90 | 10 | 671 | 40 | 0.060 | 3.3 | 4.7 | 4.7 | 22.5 | ○ |
| Example1-3 | 68 | 32 | 253 | 10 | 0.040 | 4.1 | 4.3 | 5.7 | 24.8 | ○ |
| Example1-4 | 68 | 32 | 82 | 16 | 0.195 | 3.5 | 4.6 | 5.0 | 23.3 | ○ |
| Comparative Example1-1 | 65 | 35 | 146 | NO DETECT | - | 2.7 | 4.8 | 6.3 | 30.7 | ○ |
| ComparativeExample1-2 | 68 | 32 | 102 | NO DETECT | - | 0.5 | 25.1 | 4.8 | 121.3 | ○ |
| ComparativeExample1-3 | 95 | 5 | 1300 | NO DETECT | - | - | - | - | - | × |
| ComparativeExample1-4 | 50 | 50 | 100 | NO DETECT | - | 0.8 | 14.6 | 8.7 | 126.6 | ○ |

As shown in Table 1-1, Examples 1-1 to 1-4 each have a pore size distribution with two or more different mode values (satisfy the formula (1-I)), and have high reactivity and sufficient detaching strength.

In contrast, Comparative Examples 1-1 to 1-4 do not have a pore size distribution with two or more different mode values (not satisfy the formula (1-I)), and have low reactivity.

Table 1-2

| | Powdery catalyst (%) | Amount of binder (%) | maximum mode value t1 of catalyst layer (n m) | mode value t2 of catalyst layer (n m) | $t_2/t_1$ | Reaction characteristics | | | |
|---|---|---|---|---|---|---|---|---|---|
| | | | | | | reaction rate (%) | Selectivity | reaction rate × Sselectivity | Detaching strength |
| Example1-5 | 80 | 20 | 477 | 11 | 0.023 | 40.2 | 39.9 | 1604 | ○ |
| Comparative example1-5 | 68 | 32 | 102 | NO DETECT | - | 3.3 | 24.2 | 80 | ○ |

As shown in Table 1-2, Example 1-5 has a pore size distribution with two or more different mode values (satisfy the formula (1-I)), and has good reaction characteristics (high reaction rate × selectivity) and sufficient detaching strength.
The aldehyde reaction is an equilibrium reaction and known to be difficult to balance a reaction rate and a selectivity. The reaction using the film catalyst of the first invention achieved good reaction characteristics.
In contrast, Comparative Example 1-5 does not have a pore size distribution with two or more different mode values (not satisfy the formula (1-I)), and has poor reaction characteristics (low reaction rate × selectivity).

Examples and Comparative Examples of the second invention

Preparation Example 2-1 (preparation of powdery catalyst)

A powdery catalyst containing a ternary catalytic active substance of copper-nickel-ruthenium and a synthetic zeolite carrying them was prepared as follows.
To a 1 L flask, which contained a synthetic zeolite (average particle diameter 6 μm), was added an aqueous solution of copper nitrate, nickel nitrate and ruthenium chloride prepared such that a molar ratio of metal atoms was Cu: Ni:Ru=4:1:0.01, and heated to rise a temperature with stirring. To this was added dropwise an aqueous solution of 10 % by mass sodium carbonate at 90˚C with controlling pH of the mixture to 9 to 10. The mixture was aged for one hour and filtered to separate a precipitate. The precipitate was washed with water, dried for 10 hours at 80˚C, and calcined for three hours at 600˚C to give a powdery catalyst. The resultant powdery catalyst contained 50 % by mass of metal oxide and 50 % by mass of synthetic zeolite. The powdery catalyst had a pore size distribution with a mode value $T_1$ of 515 nm as determined by mercury porosimetry.

Preparation Examples 2-2 and 2-3 (preparation of powdery catalyst)

Powdery catalysts were similarly prepared as in Preparation Example 2-1, except that in Preparation Example 2-2, an average particle diameter of a synthetic zeolite was 3 μm, and in Preparation Example 2-3, an average particle diameter of a synthetic zeolite was 3 μm and a temperature at which an aqueous solution of 10 % by mass sodium carbonate was added was 80˚C. The resultant powdery catalysts also contained a ternary catalytic active substance of copper-nickel-ruthenium and a synthetic zeolite carrying them. Mode values $T_1$ of pore size distributions of the powdery catalyst in Preparation Examples 2-2 and 2-3 were 230 nm and 258 nm, respectively, as determined by mercury porosimetry.

Preparation Example 2-4 (preparation of phenol resin)

A resole-type phenol resin was prepared as follows. In a 4 L flask, paraformaldehyde (F), phenol (P) and zinc acetate as a reaction catalyst (CAT) in a molar ratio of F/P/CAT=1.8/1.0/0.015 were stirred and reacted for 16 hours at 80˚C to give a resole-type phenol resin.
Examples and Comparative Examples will be described bellow. Examples 2-1 to 2-4 and Comparative Examples 2-1 to 2-4 describe a method for preparing film catalysts that can be used in tertiary amine preparation, and Example 2-5 and Comparative Example 2-5 describe a method for preparing film catalysts that can be used in aldehyde preparation.

Example 2-1

To a 250 ml wide-mouthed polyethylene bottle were added MIBK as a solvent, a phenol resin (PR-9480, SUMITOMO BAKELITE Co., Ltd.,) as a binder and the powdery catalyst prepared in Preparation Example 2-1 in this order. The phenol resin was added in such ratio as 20 parts by mass of involatile matter thereof to 80 parts by mass (65 g) of powdery catalyst. MIBK was added in such amount as a solid content in a mixture was 60 % by mass. Glass beads of 1 mm diameter (apparent volume: 65 ml) as a dispersing media were further added to the wide-mouthed polyethylene bottle.
The wide-mouthed polyethylene bottle was set in a paint shaker and subjected to a mixing and dispersing treatment for 30 minutes, which was a period of preparation, to give a coating composition. The paint shaker run under the following conditions: a movement stroke of 5 cm, a cycle of 12.5 strokes per a second, and a typical velocity of $0.05 \times 2(m)/[1/12.5]\ (s)=1.25$ m/s.
The coating composition was applied on both sides of a copper foil (thickness: 40 μm, basis weight: 310 g/$m^2$), which was used as a substrate, with a bar coater, and treated for 30 seconds at 130˚C in a hot air circulation-type thermostatic oven to give an intermediate for film catalyst.
The resultant intermediate for film catalyst (width: 65 mm × length: 160 mm) was processed into a corrugated sheet. The corrugated sheet and planar film catalysts having the same dimensions were laminated and rolled to form a cylindrical intermediate for film catalyst. 14 cylindrical intermediates each having an outer diameter 30 mm and a height 65 mm were prepared. These were subjected to a curing treatment for 90 minutes at 150˚C in a dryer to give film catalysts having a shape shown in Fig. 7.

In the resultant film catalyst, masses of the catalyst layer per unit area were 17.9 g/$m^2$ for single side and 35.8 g/$m^2$ for both sides. Amounts of the powdery catalyst carried per unit area were 14.3 g/$m^2$ for single side and 28.6 g/$m^2$ for both sides. In the 14 cylindrical film catalysts, an amount of the powdery catalyst carried was 8.2 g. An average thickness of the catalyst layer (single side) was 10 $\mu$m.

Example 2-2

To a 500 ml beaker were added MIBK as a solvent, a phenol resin (PR-9480, SUMITOMO BAKELITE Co., Ltd.,) as a binder and the powdery catalyst prepared in Preparation Example 2-1 in this order. The phenol resin was added in such ratio as 20 parts by mass of involatile matter thereof to 80 parts by mass (350 g) of powdery catalyst. MIBK was added in such amount as a solid content in a mixture was 60 % by mass.

The mixture was subjected to a mixing and dispersing treatment for 30 minutes, which was a period of preparation, with a desk dissolver to give a coating composition.

The coating composition was applied on a copper foil (thickness: 40 $\mu$m, basis weight: 310 g/$m^2$), which was used as a substrate, with a bar coater, and treated for 30 seconds at 130˚C in a hot air circulation-type thermostatic oven to form a catalyst layer, thereby giving an intermediate for film catalyst. Catalyst layers were formed on both sides of the copper foil by the same method.

The resultant intermediate for film catalyst (width: 65 mm × length: 150 mm) was processed into a corrugated sheet. The corrugated sheet and planar film catalysts having the same dimensions were laminated and rolled to form a cylindrical intermediate for film catalyst. 14 cylindrical intermediates each having an outer diameter 30 mm and a height 65 mm were prepared. These were subjected to a curing treatment for 90 minutes at 150˚C in a dryer to give film catalysts having a shape shown in Fig. 7.

In the resultant film catalyst, masses of the catalyst layer per unit area were 18.0 g/$m^2$ for single side and 36.0 g/$m^2$ for both sides. Amounts of the powdery catalyst carried per unit area were 14.4 g/$m^2$ for single side and 28.8 g/$m^2$ for both sides. In the 14 cylindrical film catalysts, an amount of the powdery catalyst carried was 8.0 g. An average thickness of the catalyst layer (single side) was 12 $\mu$m.

Example 2-3

To a 250 ml wide-mouthed polyethylene bottle were added MIBK as a solvent, the phenol resin as a binder prepared in Preparation Example 2-4 and the powdery catalyst prepared in Preparation Example 2-2 in this order. The phenol resin was added in such ratio as 32 parts by mass of involatile matter thereof to 68 parts by mass (65 g) of powdery catalyst. MIBK was added in such amount as a solid content in a mixture was 60 % by mass. Zirconia beads of 2 mm diameter (apparent volume: 65 ml) as a dispersing media were further added to the wide-mouthed polyethylene bottle.

The wide-mouthed polyethylene bottle was set in a paint shaker and subjected to a mixing and dispersing treatment for 110 minutes, which was a period of preparation, to give a coating composition. A typical velocity was same to Example 2-1, 1.25m/s.

The coating composition was applied on a copper foil (thickness: 40 $\mu$m, basis weight: 310 g/$m^2$), which was used as a substrate, with a bar coater, and treated for 30 seconds at 130˚C in a hot air circulation-type thermostatic oven to form a catalyst layer, thereby giving an intermediate for film catalyst. Catalyst layers were formed on both sides of the copper foil by the same method.

The resultant intermediate for film catalyst (width: 65 mm × length: 150 mm) was processed into a corrugated sheet. The corrugated sheet and planar film catalysts having the same dimensions were laminated and rolled to form a cylindrical intermediate for film catalyst. 12 cylindrical intermediates each having an outer diameter 30 mm and a height 65 mm were prepared. These were subjected to a curing treatment for 90 minutes at 150˚C in a dryer to give film catalysts having a shape shown in Fig. 7.

In the resultant film catalyst, masses of the catalyst layer per unit area were 9.6 g/$m^2$ for single side and 19.3 g/$m^2$ for both sides. Amounts of the powdery catalyst carried per unit area were 6.6 g/$m^2$ for single side and 13.2 g/$m^2$ for both sides. In the 12 cylindrical film catalysts, an amount of the powdery catalyst carried was 3.1g. An average thickness of the catalyst layer (single side) was 4 $\mu$m.

Example 2-4

To a 1L vessel were added MIBK as a solvent, a phenol resin (PR-9480, SUMITOMO BAKELITE Co., Ltd.,)

as a binder and the powdery catalyst prepared in Preparation Example 2-3 in this order. The phenol resin was added in such ratio as 35 parts by mass of involatile matter thereof to 65 parts by mass (350 g) of powdery catalyst. MIBK was added in such amount as a solid content in a mixture was 60 % by mass.

The mixture was subjected to a pretreatment of mixing and stirring for 10 minutes with a desk dissolver, and checked visually. The mixture confirmed not to contain an aggregate of powdery catalyst was subjected to a mixing and stirring treatment for 30 minutes to give a coating composition. The coating composition was subjected to a mixing and dispersing treatment for 60 minutes, which was a period of preparation, with a basket mill [ASADA IRON WORKS.CO., LTD., model SS-L, a 150ml basket filled with titania beads of 1.4 mm diameter (apparent volume: 120 ml)] at 3000 rpm, or 7 m/s of a typical velocity or a peripheral speed.

The coating composition was applied on both sides of a copper foil (thickness: 40 μm, basis weight: 310 g/m2), which was used as a substrate, with a bar coater, and treated for 30 seconds at 130˚C in a hot air circulation-type thermostatic oven to give an intermediate for film catalyst having a shape shown in Fig. 7.

The resultant intermediate for film catalyst (width: 65 mm × length: 150 mm) was processed into a corrugated sheet. The corrugated sheet and planar film catalysts having the same dimensions were laminated and rolled to form a cylindrical intermediate for film catalyst. 15 cylindrical intermediates each having an outer diameter 30 mm and a height 65 mm were prepared. These were subjected to a curing treatment for 90 minutes at 150˚C in a dryer to give film catalysts having a shape shown in Fig. 7.

In the resultant film catalyst, masses of the catalyst layer per unit area were 29.2 $g/m^2$ for single side and 58.4 $g/m^2$ for both sides. Amounts of the powdery catalyst carried per unit area were 19.0 $g/m^2$ for single side and 38.0 $g/m^2$ for both sides. In the 15 cylindrical film catalysts, an amount of the powdery catalyst carried was 11.1 g. An average thickness of the catalyst layer (single side) was 12 μm. Example 2-5

To a 250 ml wide-mouthed polyethylene bottle were added MIBK as a solvent, a phenol resin (PR-9480, SUMITOMO BAKELITE Co., Ltd.,) as a binder and the powdery catalyst prepared in Preparation Example 2-1 in this order. The phenol resin was added in such ratio as 20 parts by mass of involatile matter thereof to 80 parts by mass (65 g) of powdery catalyst. MIBK was added in such amount as a solid content in a mixture was 60 % by mass. Glass beads of 1 mm diameter (apparent volume: 65 ml) as a dispersing media were further added to the wide-mouthed polyethylene bottle.

The wide-mouthed polyethylene bottle was set in a paint shaker and subjected to a mixing and dispersing treatment for 30 minutes, which was a period of preparation, to give a coating composition.

The coating composition was applied on both sides of a copper foil (thickness: 40 μm, basis weight: 310 $g/m^2$), which was used as a substrate, with a bar coater, and treated for 30 seconds at 130˚C in a hot air circulation-type thermostatic oven to give an intermediate for film catalyst (the same intermediate as in Example 2-1).

The resultant intermediate for film catalyst (130 mm × 840 mm) was processed into a planar and corrugated sheet. The corrugated and planar sheets were alternatively laminated and put in a stainless-steel cylindrical reactor having an inner diameter 21.4 mm and a length 390 mm (Fig. 9). The reactor was subjected to a curing treatment for 90 minutes at 150˚C in a hot air circulation-type thermostatic oven to give a film catalyst.

In the resultant film catalyst, masses of the catalyst layer per unit area were 17.9 $g/m^2$ for single side and 35.8 $g/m^2$ for both sides. Amounts of the powdery catalyst carried per unit area were 14.3 $g/m^2$ for single side and 28.6 $g/m^2$ for both sides. In the film catalyst put in the cylindrical reactor, an amount of the powdery catalyst in the film catalyst was 3.1 g. An average thickness of the catalyst layer (single side) was 10 μm.

Comparative Example 2-1

To a 15 L vessel were added MIBK as a solvent, a phenol resin (PR-9480, SUMITOMO BAKELITE Co., Ltd.,) as a binder and the powdery catalyst prepared in Preparation Example 2-3 in this order. The phenol resin was added in such ratio as 35 parts by mass of involatile matter thereof to 65 parts by mass (4500 g) of powdery catalyst. MIBK was added in such amount as a solid content in a mixture was 60 % by mass.

The mixture was subjected to a pretreatment of mixing and stirring for 30 minutes with a desk dissolver (number of rotations: 800 rpm, peripheral speed: 4 m/s) to give a coating composition, and the resultant coating composition was then subjected to a mixing and dispersing treatment for 325 minutes, which was a period of preparation, with a basket mill [ASADA IRON WORKS.CO.,LTD., model SS-3, a 1 L basket filled with titania beads of 1.4 mm diameter (apparent volume: 800 ml, 1900 g)] at 1500 rpm, or 4.8 m/s of typical velocity or peripheral speed.

The coating composition was applied on both sides of a copper foil (thickness: 40 μm, basis weight: 310 $g/m^2$), which was used as a substrate, with a bar coater, and treated for 30 seconds at 130˚C in a hot air circulation-type thermostatic oven to give an intermediate for film catalyst.

The resultant intermediate for film catalyst (width: 65 mm × length: 150 mm) was processed into a corrugated sheet. The corrugated sheet and planar film catalysts having the same dimensions were laminated and rolled to form a cylindrical intermediate for film catalyst. 12 cylindrical intermediates each having an outer diameter 30 mm and a height 65 mm were prepared. These were subjected to a curing treatment for 90 minutes at 150˚C in a dryer to give film catalysts having a shape shown in Fig. 7.

In the resultant film catalyst, masses of the catalyst layer per unit area were 14.9 $g/m^2$ for single side and 29.7 $g/m^2$ for both sides. Amounts of the powdery catalyst carried per unit area were 9.7 $g/m^2$ for single side and 19.4 $g/m^2$ for both sides. In the 12 cylindrical film catalysts, an amount of the powdery catalyst carried was 4.5 g. An average thickness of the catalyst layer (single side) was 6 $\mu$m.

Comparative Example 2-2

To a 250 ml wide-mouthed polyethylene bottle were added MIBK as a solvent, a phenol resin (PR-9480, SUMITOMO BAKELITE Co., Ltd.,) as a binder and the powdery catalyst prepared in Preparation Example 2-2 in this order. The phenol resin was added in such ratio as 32 parts by mass of involatile matter thereof to 68 parts by mass (65 g) of powdery catalyst. MIBK was added in such amount as a solid content in a mixture was 60 % by mass. Zirconia beads of 2 mm diameter (apparent volume: 65 ml) as a dispersing media were further added to the polyethylene bottle.

The wide-mouthed polyethylene bottle was set in a paint shaker and subjected to a mixing and dispersing treatment for 180 minutes, which was a period of preparation, to give a coating composition. A typical velocity was same to Example 2-1, 1.25 m/s.

The coating composition was applied on both sides of a copper foil (thickness: 40 $\mu$m, basis weight: 310 $g/m^2$), which was used as a substrate, with a bar coater, and treated for 30 seconds at 130˚C in a hot air circulation-type thermostatic oven to give an intermediate for film catalyst.

The resultant intermediate for film catalyst (width: 65 mm $\times$ length: 150 mm) was processed into a corrugated sheet. The corrugated sheet and planar film catalysts having the same dimensions were laminated and rolled to form a cylindrical intermediate for film catalyst. 12 cylindrical intermediates each having an outer diameter 30 mm and a height 65 mm were prepared. These were subjected to a curing treatment for 180 minutes at 210˚C in a dryer to give film catalysts having a shape shown in Fig. 7.

In the resultant film catalyst, masses of the catalyst layer per unit area were 12.9 $g/m^2$ for single side and 25.9 $g/m^2$ for both sides. Amounts of the powdery catalyst carried per unit area were 8.8 $g/m^2$ for single side and 17.6 $g/m^2$ for both sides. In the 12 cylindrical film catalysts, an amount of the powdery catalyst carried was 4.1g. An average thickness of the catalyst layer (single side) was 6 $\mu$m.

Comparative Example 2-3

To a 250 ml wide-mouthed polyethylene bottle were added MIBK as a solvent, a phenol resin (PR-9480, SUMITOMO BAKELITE Co., Ltd.,) as a binder and the powdery catalyst prepared in Preparation Example 2-1 in this order. The phenol resin was added in such ratio as 5 parts by mass of involatile matter thereof to 95 parts by mass (65 g) of powdery catalyst. MIBK was added in such amount as a solid content in a mixture was 60 % by mass. Glass beads of 1 mm diameter (apparent volume: 65 ml) as a dispersing media were further added to the wide-mouthed polyethylene bottle.

The wide-mouthed polyethylene bottle was set in a paint shaker and subjected to a mixing and dispersing treatment for 5 minutes, which was a period of preparation, to give a coating composition. A typical velocity was same to Example 2-1, 1.25 m/s. The resultant coating composition contained many particles (flocculants).

The coating composition was applied on both sides of a copper foil (thickness: 40 $\mu$m, basis weight: 310 $g/m^2$), which was used as a substrate, with a bar coater, and treated for 30 seconds at 130˚C in a hot air circulation-type thermostatic oven to give an intermediate for film catalyst.

The resultant intermediate for film catalyst (width: 65 mm $\times$ length: 150 mm) was processed into a corrugated sheet. The corrugated sheet and planar film catalysts having the same dimensions were laminated and rolled to form a cylindrical intermediate for film catalyst. 14 cylindrical intermediates each having an outer diameter 30 mm and a height 65 mm were prepared. These were subjected to a curing treatment for 180 minutes at 210˚C in a dryer to give film catalysts having a shape shown in Fig. 7.

In the resultant film catalyst, masses of the catalyst layer per unit area were 9.0 $g/m^2$ for single side and 18.0 $g/m^2$ for both sides. Amounts of the powdery catalyst carried per unit area were 8.6 $g/m^2$ for single side and 17.2 $g/m^2$ for both sides. In the 14 cylindrical film catalysts, an amount of the powdery catalyst carried was 4.6 g. An average thickness of the catalyst layer (single side) was 8 $\mu$m. The catalyst layer of the resultant film catalyst was very fragile and had poor strength. Although the resultant film catalyst was subjected to the evaluation for reaction characteristics as described below, it could not be properly evaluated as a catalyst for fixed-bed reaction due to much dropout of the catalyst layer.

Comparative Example 2-4

To a 250 ml wide-mouthed polyethylene bottle were added MIBK as a solvent, a phenol resin (PR-9480, SUMITOMO BAKELITE Co., Ltd.,) as a binder and the powdery catalyst prepared in Preparation Example 2-3 in this order. The phenol resin was added in such ratio as 50 parts by mass of involatile matter thereof to 50 parts by mass (65 g) of powdery catalyst. MIBK was added in such amount as a solid content in a mixture was 50 % by mass. Zirconia beads of 2 mm diameter (apparent volume: 65 ml) as a dispersing media were further added to the wide-mouthed polyethylene bottle.
The wide-mouthed polyethylene bottle was set in a paint shaker and subjected to a mixing and dispersing treatment for 250 minutes, which was a period of preparation, to give a coating composition. A typical velocity was same to Example 2-1, 1.25m/s.
The coating composition was applied on both sides of a copper foil (thickness: 40 μm, basis weight: 310 g/$m^2$), which was used as a substrate, with a bar coater, and treated for 30 seconds at 130˚C in a hot air circulation-type thermostatic oven to give an intermediate for film catalyst.
The resultant intermediate for film catalyst (width: 65 mm × length: 150 mm) was processed into a corrugated sheet. The corrugated sheet and planar film catalysts having the same dimensions were laminated and rolled to form a cylindrical intermediate for film catalyst. 13 cylindrical intermediates each having an outer diameter 30 mm and a height 65 mm were prepared. These were subjected to a curing treatment for 90 minutes at 150˚C in a dryer to give film catalysts having a shape shown in Fig. 7.
In the resultant film catalyst, masses of the catalyst layer per unit area were 15.9 g/$m^2$ for single side and 31.6 g/$m^2$ for both sides. Amounts of the powdery catalyst carried per unit area were 7.9 g/$m^2$ for single side and 15.8 g/$m^2$ for both sides. In the 13 cylindrical film catalysts, an amount of the powdery catalyst carried was 4.0 g. An average thickness of the catalyst layer (single side) was 11 μm.

Comparative Example 2-5

To a 250 ml wide-mouthed polyethylene bottle were added MIBK as a solvent, a phenol resin (PR-9480, SUMITOMO BAKELITE Co., Ltd.,) as a binder and the powdery catalyst prepared in Preparation Example 2-2 in this order. The phenol resin was added in such ratio as 32 parts by mass of involatile matter thereof to 68 parts by mass (65 g) of powdery catalyst. MIBK was added in such amount as a solid content in a mixture was 60 % by mass. Zirconia beads of 2 mm diameter (apparent volume: 65 ml) as a dispersing media were further added to the wide-mouthed polyethylene bottle.
The wide-mouthed polyethylene bottle was set in a paint shaker and subjected to a mixing and dispersing treatment for 180 minutes, which was a period of preparation, to give a coating composition.
The coating composition was applied on both sides of a copper foil (thickness: 40 μm, basis weight: 310 g/$m^2$), which was used as a substrate, with a bar coater, and treated for 30 seconds at 130˚C in a hot air circulation-type thermostatic oven to give an intermediate for film catalyst (the same intermediate as in Comparative Example 2-2).
The resultant intermediate for film catalyst (130 mm × 840 mm) was processed into a planar or corrugated sheet. The corrugated and planar sheets were alternatively laminated and put in a stainless-steel cylindrical reactor having an inner diameter 21.4 mm and a length 390 mm (Fig. 9). The reactor was subjected to a curing treatment for 90 minutes at 150˚C in a hot air circulation-type thermostatic oven to give a film catalyst.
In the resultant film catalyst, masses of the catalyst layer per unit area were 12.9 g/$m^2$ for single side and 25.9 g/$m^2$ for both sides. Amounts of the powdery catalyst carried per unit area were 8.8 g/$m^2$ for single side and 17.6 g/$m^2$ for both sides. In the 12 cylindrical film catalysts, an amount of the powdery catalyst carried was 1. 9g. An average thickness of the catalyst layer (single side) was 6 μm.

(Evaluation for reaction characteristics 2-1)

Cylindrical film catalysts prepared in Examples 2-1 to 2-4 and Comparative Examples 2-1 to 2-4 each have vertical flow passages across the length of cylinder, which passage has a cross-section area of about 0.1 $cm^2$.
In a glass reactor having an inner diameter of 130 mm, the bottom surface of which was a glass filter (2G), the cylindrical film catalyst and 1000 g of dodecyl alcohol (KALCOL 2098, KAO CORPORATION) as a raw material were placed. The catalyst was reductively activated. To the reactor were supplied a hydrogen gas and dimethyl amine with keeping a steady state at 220˚C.
Samples were repeatedly collected from the reactor over time and analyzed by gas chromatography. A concentration change of dodecyl alcohol to a reaction time, a mass of dodecyl alcohol used in the reaction M=1000 [g], an amount of powdery catalyst in the film catalyst used in the reaction G (g) were used to calculate a reaction rate constant k [/Hr·($m^2$/kg-ROH)] by the calculation formula below.

$$k = \frac{-\ln(C2/C2)}{(\text{Time }2-\text{Time }1)\cdot(G/M\times 100)} \quad [/Hr\cdot\%]$$

wherein, Time1 and Time2 (Hr) are sampling points of time [Time2>Time1]; and C1 and C2 (%) are concentrations of dodecyl alcohol at the points of Time1 and Time2 , respectively.

The reaction was considered as had ended when a concentration of dodecyl alcohol reached to 1 %, and those were also determined: TIME (Hr), a reaction time from the reaction start to the reaction end; M2 (%), a concentration of side-product, dilaurylmonomethylamine, at the reaction end as determined by gas chromatography; and TIME × M2, [a reaction time × a concentration of side-product]. Results are shown in Table 2-1 below.

The index, TIME × M2, is introduced for evaluating the catalyst, because a catalyst resulting in a shorter reaction time and a smaller amount of side-product in the reaction is considered as having better reaction characteristics. A catalyst having a smaller value of the index exhibits better reaction characteristics. In Table 2-1, the total of content percentages of the powdery catalyst and the binder is 100 % by mass. In the Table 2-1, a film strength is evaluated as a detaching strength. × refers generation of detachment of a catalyst layer and a cloudy reaction mixture due to the detached catalyst layer. O refers no visually recognizable detachment and an unclouded reaction mixture after evaluation of the reaction.

(Evaluation for reaction characteristics 2-2)

To the film catalyst prepared in Example 2-5 put in the cylindrical reactor was flowed dodecyl alcohol (KALCOL 2098, KAO CORPORATION) at a flow rate 73.9 g/Hr and a hydrogen gas at a flow rate 33.6 NL/Hr to reductively activate the catalyst.

Then, hydrogen gas supply was stopped. To the cylindrical reactor were supplied an $N_2$ gas at 9 NL/Hr and dodecyl alcohol at 73.9 g/Hr with increasing a temperature to 220°C. The reaction system was hold at the temperature to continuously progress dehydrogenation. A reaction pressure is an ambient pressure.

A sample taken from the reaction mixture was analyzed by gas chromatography. A concentration of dodecyl alcohol was 58.4%, a concentration of aldehyde produced was 16.1 %. Considering a concentration of dodecyl alcohol used as a raw material was 98.6 %, the followings were calculated using these values:

reaction rate (%) = concentration of dodecyl alcohol as a raw material - concentration of dodecyl alcohol after the reaction

=98.6-58.4=40.2 (%)

selectivity (%) = concentration of aldehyde after the reaction (%) / reaction rate (%)× 100

=16.1/40.2×100=39.9 (%).

Results are shown in Table 2-2 below.

In Table 2-2, the total of content percentages of the powdery catalyst and the binder is 100 % by mass. Detaching strength in the Table 2-2 refers the same meaning as in Table 2-1.

The index, reaction rate × selectivity, is introduced for evaluating the catalyst, because a catalyst resulting in a higher reaction rate and a higher selectivity in the reaction is considered as having better reaction characteristics. A catalyst having a larger value of the index exhibits better reaction characteristics. The reason of using a different index from the index in "Evaluation for reaction characteristics 2-1" is that the reaction system differs from 2-1 and cannot be evaluated by TIME × M2.

(Evaluation for reaction characteristics 2-3)

Similarly as in Evaluation for reaction characteristics 2-2, to the film catalyst prepared in Comparative Example 2-5 put in the cylindrical reactor was flowed dodecyl alcohol (KALCOL 2098, KAO CORPORATION) at a flow rate 73.9 g/Hr and a hydrogen gas at a flow rate 33.6 NL/Hr to reductively activate the catalyst.
Then, hydrogen gas supply was stopped. To the cylindrical reactor were supplied an $N_2$ gas at 9 NL/Hr and dodecyl alcohol at 73.9 g/Hr with increasing a temperature to 220°C. The reaction system was hold at the temperature to continuously progress dehydrogenation. A reaction pressure is an ambient pressure.
A sample taken from the reaction mixture was analyzed by gas chromatography. A concentration of dodecyl alcohol was 95.3 %, and a concentration of aldehyde produced was 0.8 %. Considering a concentration of dodecyl alcohol used as a raw material was 98.6 %, the followings were calculated using these values:

reaction rate (%) = concentration of dodecyl alcohol as a raw material - concentration of dodecyl alcohol after the reaction

$$=98.6-95.3=3.3\ (\%)$$

selectivity (%) = concentration of aldehyde after the reaction (%) / reaction rate (%) × 100

$$=0.8/3.3\times100=24.2\ (\%).$$

Results are shown in Table 2-2 below.

(Method for measuring a pore size distribution)

A pore size distribution was measured in accordance with the method (mercury porosimetry) described, for example, in "Busshitsu no kinousei (functionality of material), Jikken Kagaku Kouza (Experimental Chemistry) the 4th ed., ed by the Chemical Society of Japan, Maruzen, Tokyo, vol. 12, p.486". More specifically, a pore size distribution can be measured with a special measuring apparatus such as a mercury intrusion porosimeter (Poresizer9320) manufactured by Shimadsu Corporation.
The film catalyst, which was previously measured for a basis weight thereof and a basis weight and an area of copper foil before application of the coating composition, was measured for a mass of the catalyst layer. A pore size distribution of the catalyst layer was measured by mercury porosimetry. In mercury porosimetry, a pore size was calculated by the calculation formula below:

$$D=-4\gamma\cos\theta/P$$

wherein, D represents a pore size, $\gamma$ represents a mercury surface tension, $\theta$ represents a contact angle and P represents a pressure.
In the measurement, a mercury surface tension was 482.536 dyn/cm, a contact angle used was 130°, and a mercury pressure was from 0 to 30000 psia.
Based on the principle described above, a pore size distribution is determined by putting pressure upon mercury and measuring a volume of mercury intruding pores, or a volume V, with gradually varying the pressure, plotting a pore size D calculated according to the calculation formula against a volume, finding a derivative dV/d(logD) of the plotted curve, and making a graph with a vertical axis of the derivative against a horizontal axis of the pore size D (see, Fig. 4-2). This method is mercury porosimetry. A pore size distribution was measured in the range from 6 nm to 10000 nm of pore size. In Figs. 4-2 and 5, a unit (ml) in the volume refers an amount of intruding mercury and (g) refers a mass of sample of the catalyst layer used in the measurement. "NO DETECT" means that a mode value cannot be visually recognized in the graph.

In pore size distributions having different mode values (peaks of the curve plotted in the graph) of the catalyst layer measured by the method, a mode value in a pore size distribution which value was maximum was set to a mode value $t_1$, and a mode value smaller than $t_1$ but having a largest peak among the other mode values was set to a mode value $t_2$.

Table 2-1

| | Powdery catalyst (%) | Amount of binder (%) | time·V·P/G (m·L/kg) | Maximum mode value T1 of the catalyst powder (nm) | Maximum mode value t1 of the catalyst layer (nm) | Mode value t2 of the catalyst layer (nm) | $t_1/T_1$ | $t_2/t_1$ | Reaction characteristics | | | | |
|---|---|---|---|---|---|---|---|---|---|---|---|---|---|
| | | | | | | | | | Reactivity k (/Hr·%) | Reaction time TIME (Hr) | side-product M2 (%) | TIME × M2 | Detaching strength |
| Example 2-1 | 80 | 20 | 1080 | 515 | 477 | 11 | 0.93 | 0.02 | 3.8 | 2.4 | 8.1 | 19.4 | ○ |
| Example 2-2 | 80 | 20 | - | 515 | 974 | 13 | 1.89 | 0.01 | 2.5 | 3.2 | 7.9 | 24.9 | ○ |
| Example 2-3 | 68 | 32 | 3366 | 230 | 253 | 10 | 1.10 | 0.04 | 4.1 | 4.3 | 5.7 | 24.8 | ○ |
| Example 2-4 | 65 | 35 | 1685 | 258 | 195 | NO DETECT | 0.75 | - | 1.7 | 3.8 | 10.6 | 40.3 | ○ |
| Comparative example 2-1 | 65 | 35 | 6490 | 258 | 100 | NO DETECT | 0.39 | - | 0.9 | 11.0 | 13.9 | 152.7 | ○ |
| Comparative example 2-2 | 68 | 32 | 5508 | 230 | 102 | NO DETECT | 0.44 | - | 0.5 | 25.1 | 4.8 | 121.3 | ○ |
| Comparative example 2-3 | 95 | 5 | 214 | 515 | 1300 | NO DETECT | 2.52 | - | - | - | - | - | × |
| Comparative example 2-4 | 50 | 50 | 5625 | 258 | 100 | NO DETECT | 0.39 | - | 0.8 | 14.6 | 8.7 | 126.6 | ○ |

As shown in Table 2-1, in each of Examples 2-1 to 2-4, the mode value $T_1$ of pore size distribution of the powdery catalyst and the mode value $t_1$ of pore size distribution of the catalyst layer in the film catalyst satisfy the formula (2-I). These catalysts had high reactivity, produced a small amount of side-product, and had good characteristics and sufficient detaching strength.

In contrast, in Comparative Examples 2-1 to 2-4, the mode value $T_1$ of pore size distribution of the powdery catalyst and the mode value $t_1$ of pore size distribution of the catalyst layer in the film catalyst differs largely each other. These catalysts had low reactivity and produced a large amount of side-product.

Table 2-2

| | Powdery catalyst (%) | Amount of binder (%) | time·V·P/G (m·L/kg) | Maximum mode value T1 of the catalyst powder (nm) | Maximum mode value t1 of the catalyst layer (nm) | Mode value t2 of the catalyst layer (nm) | $t_1/T_1$ | $t_2/t_1$ | Reaction characteristics | | | |
|---|---|---|---|---|---|---|---|---|---|---|---|---|
| | | | | | | | | | Reactivity (%) | Selectivity (%) | Reactivity × Selectivity | Detaching strength |
| Example 2-5 | 80 | 20 | 1080 | 515 | 477 | 11 | 0.93 | 0.02 | 40.2 | 39.9 | 1604 | ○ |
| Comparative example 2-5 | 68 | 32 | 5508 | 230 | 102 | NO DETECT | 0.44 | - | 3.3 | 24.2 | 80 | ○ |

As shown in Table 2-2, in Example 2-5, the mode value $T_1$ of pore size distribution of the powdery catalyst and the mode value $t_1$ of pore size distribution of the catalyst layer in the film catalyst satisfy the formula (2-I). The catalyst had good reaction characteristics (high reaction rate × selectivity) and sufficient detaching strength.
An aldehyde reaction is equilibrium and known to be difficult to balance a reaction rate and a selectivity. The reaction using the film catalyst of the second invention achieved good reaction characteristics.
In contrast, in Comparative Example 2-5, the mode value $T_1$ of pore size distribution of the powdery catalyst and the mode value $t_1$ of pore size distribution of the catalyst layer in the film catalyst did not satisfy the formula (2-I). The catalyst had poor reaction characteristics (low reaction rate × selectivity).

**Claims**

1. A film catalyst, comprising a substrate and a catalyst layer, formed on the substrate, comprising a powdery catalyst and a binder and having a pore size distribution with two or more different mode values as determined by mercury porosimetry.

2. The film catalyst according to claim 1, wherein the maximum mode value $t_1$ and a mode value $t_2$ different from the mode value $t_1$, in the two or more different mode values as determined by mercury porosimetry, satisfy a relationship represented by the formula (1-I):

$$t_2 \leq 0.2 \times t_1 \quad (1\text{-}I)$$

3. The film catalyst according to claim 2, wherein the mode value $t_1$ is 20 nm to 250 μm.

4. The film catalyst according to claim 2 or 3, wherein the mode value $t_2$ is 4 to 50nm.

5. The film catalyst according to any of claims 1 to 4, wherein the powdery catalyst contained in the catalyst layer is a catalytic active substance carried on a porous material.

6. The film catalyst according to any of claims 1 to 5, wherein the catalyst layer has a thickness of not more than 100 μm.

7. The film catalyst according to any of claims 1 to 6, which has a honeycomb structure.

8. The film catalyst according to any of claims 1 to 7, which is used as a catalyst for gas-liquid reaction.

9. A method for producing a film catalyst comprising a substrate and a catalyst layer formed on the substrate, comprising steps:

   preparing a coating composition comprising a powdery catalyst, a binder and a solvent; and
   forming the catalyst layer on the substrate with the coating composition to obtain a catalyst intermediate, wherein the resultant film catalyst satisfies the formula (2-I):

$$0.55\ T_1 < t_1 < 2\ T_1 \quad (2\text{-}I)$$

   wherein
   $T_1$: the maxim mode value of a pore size distribution of the powdery catalyst itself, used as a raw material contained in the catalyst layer of the film catalyst, as determined by mercury porosimetry and
   $t_1$: the maxim mode value of a pore size distribution of the catalyst layer of the film catalyst, as determined by mercury porosimetry.

10. The method for producing the film catslyst according to claim 9, wherein the catalyst layer of the film catalyst has a pore size distribution further having a mode value $t_2$ differing from the $t_1$ as determined by mercury porosimetry and satisfying the formula:

$$t_2 \leq 0.2 \times t_1$$

**11.** The method for producing the film catslyst according to claim 10, wherein the mode value $t_2$ is 4 to 50 nm.

**12.** The method for producing the film catslyst according to any of claims 9 to 11, wherein the coating composition is prepared with a media-type mill.

**13.** The method for producing the film catslyst according to any of claims 9 to 12, wherein the coating composition is prepared under the conditions satisfying the formula:

$$500 < \mathrm{time} \cdot V \cdot P / G < 5000 \quad (\mathrm{m} \cdot \mathrm{L/kg})$$

wherein, time (sec.) is a period of preparation in the media-type mill, V (m/sec.) is a typical velocity, P (L) is an apparent volume of a media used in preparation and G (kg) is a blended mass of the coating composition.

**14.** The method for producing the film catslyst according to any of claims 9 to 13, further comprising drying and/or curing the catalyst intermediate.

**15.** The method for producing the film catslyst according to claim 14, further comprising forming the catalyst intermediate in a shape and heating it after the drying and/or curing.

**16.** The method for producing the film catslyst according to claim 15, wherein the forming step is to form the catalyst intermediate in a honeycomb structure.

**17.** The method for producing the film catslyst according to any of claims 9 to 16, wherein the powdery catalyst contained in the coating composition is a catalytic active substance carried on a porous material.

**18.** The method for producing the film catslyst according to any of claims 9 to 17, wherein the binder is contained in the coating composition in an amount of 10 to 40 % by mass.

**19.** The method for producing the film catslyst according to any of claims 9 to 18, wherein a thickness of the catalyst layer of the film catalyst is not more than 100 μm.

**20.** The method for producing the film catslyst according to any of claims 9 to 19, wherein the film catalyst is used as a catalyst for a gas-liquid reaction.

**21.** Use of the film catalyst according to any of claims 1 to 8 or prepared by the method for production according to any of claims 9 to 20 as a catalyst for a gas-liquid reaction.

**22.** A method for conducting a gas-liquid reaction with the film catalyst according to any of claims 1 to 8 or prepared by the method for production according to any of claims 9 to 20.

Fig. 1

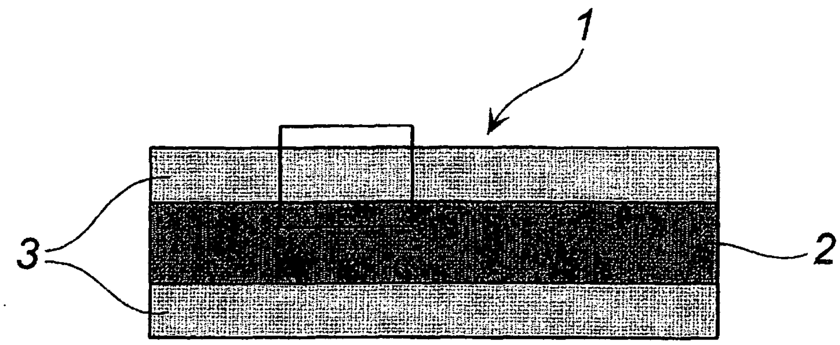


Fig. 2

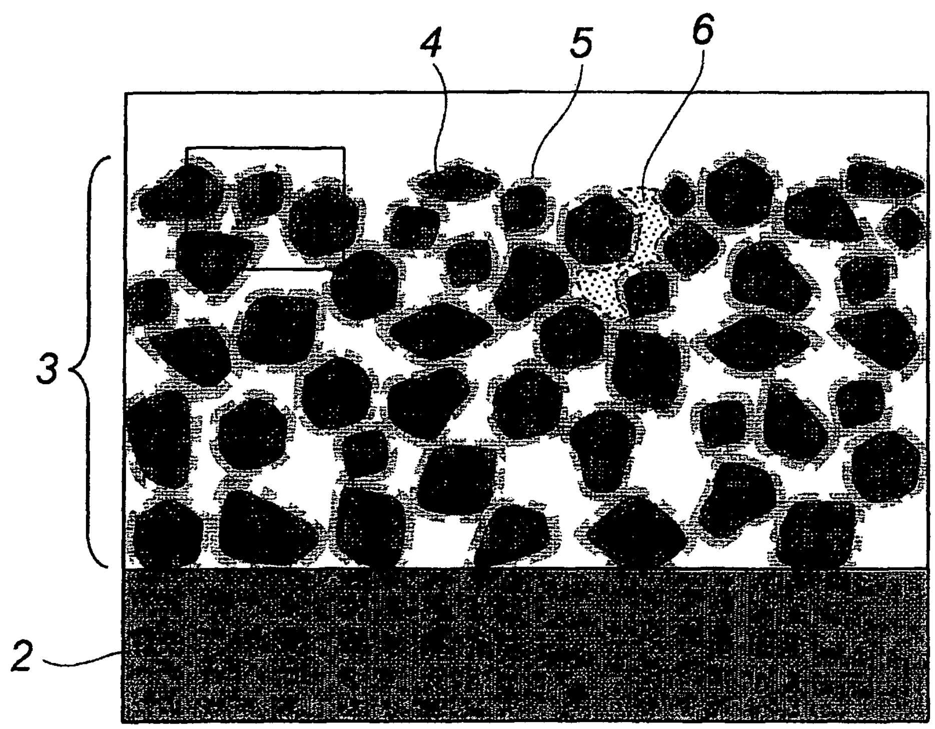

Fig. 3

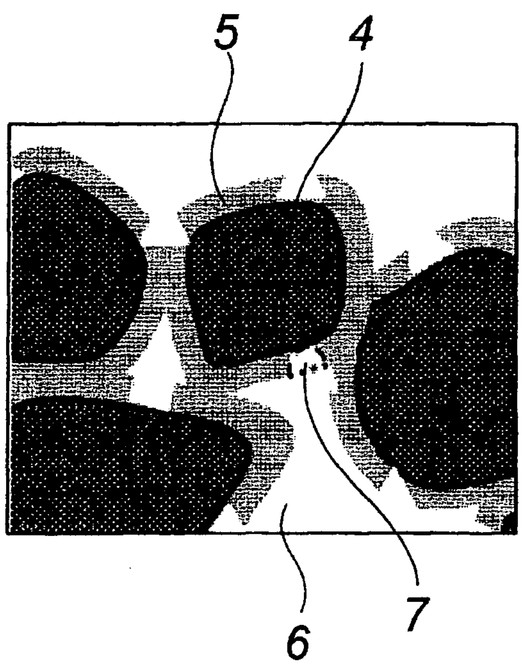

Fig. 4-1

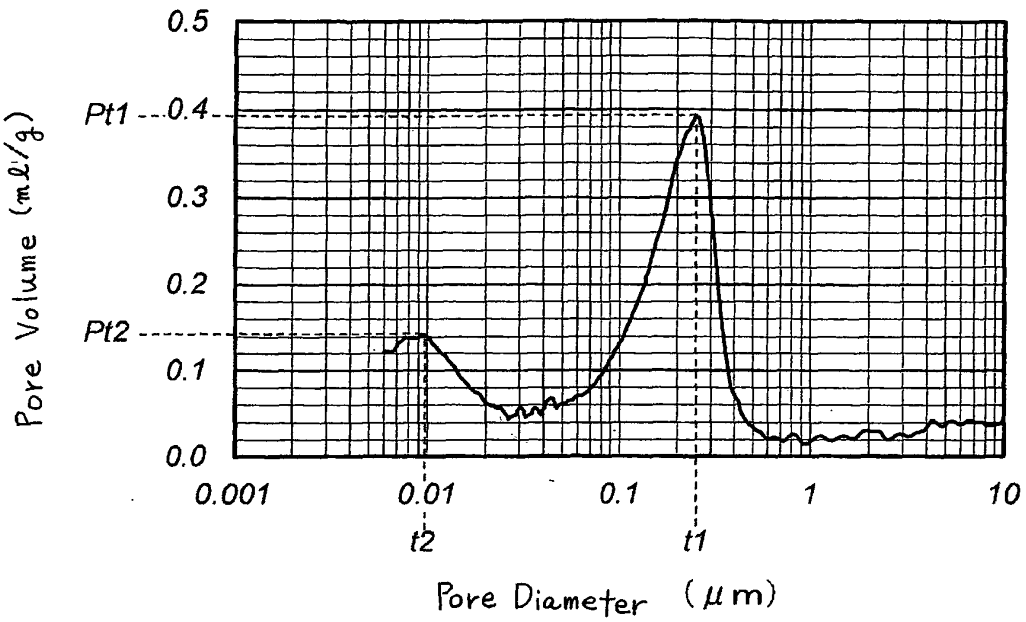
0.5
Pt1
0.4
0.3
0.2
Pt2
0.1
0.0
Pore Volume (mℓ/g)
0.001
0.01
0.1
1
10
t2
t1
Pore Diameter (μm)

Fig. 4-2

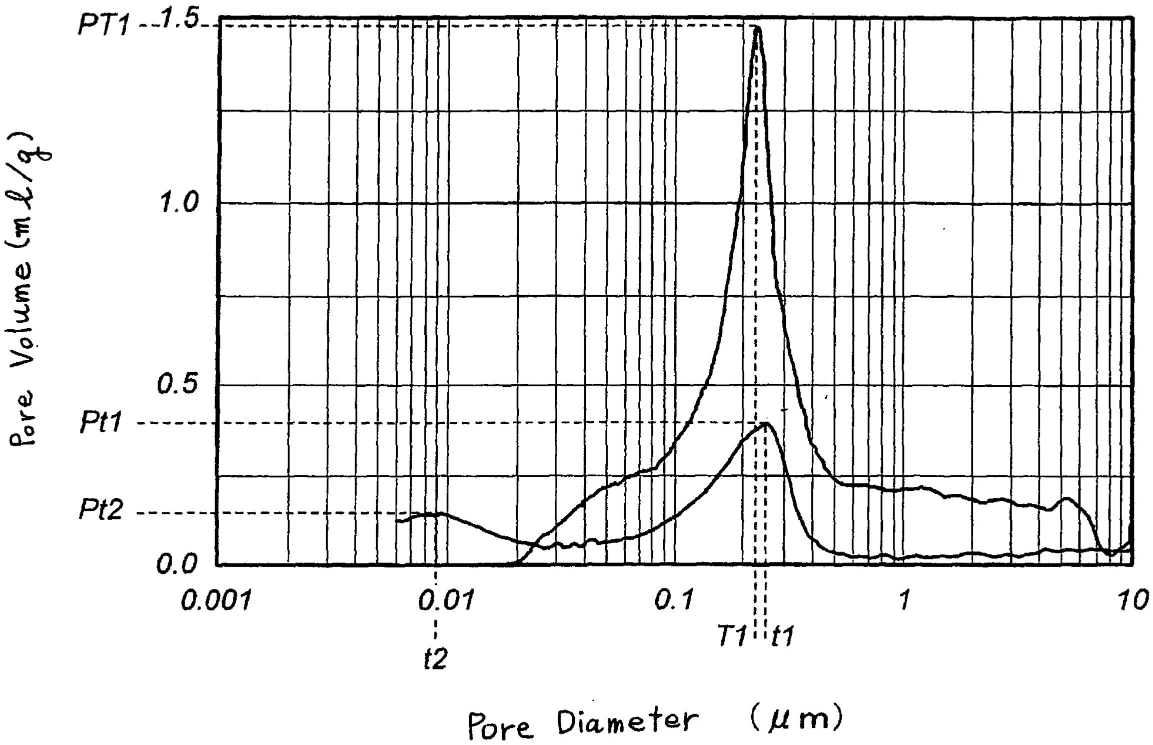
PT1
1.5
1.0
0.5
Pt1
Pt2
0.0
Pore Volume (ml/g)
0.001
0.01
0.1
1
10
t2
T1
t1
Pore Diameter (μm)

Fig. 5

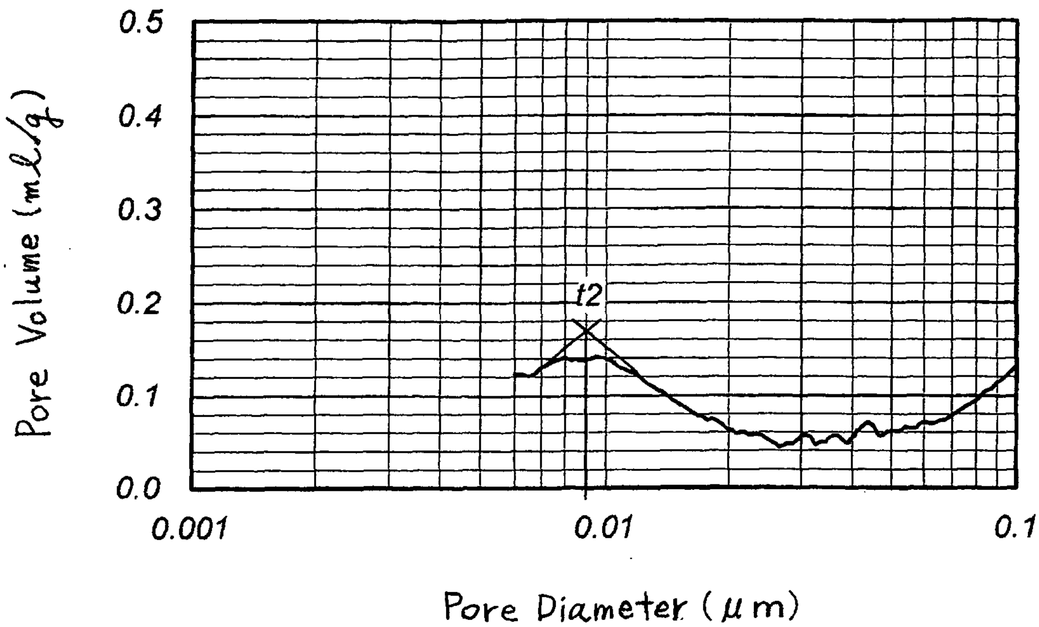
0.5
0.4
0.3
0.2
0.1
0.0
Pore Volume (ml/g)
t2
0.001
0.01
0.1
Pore Diameter (μm)

Fig. 6

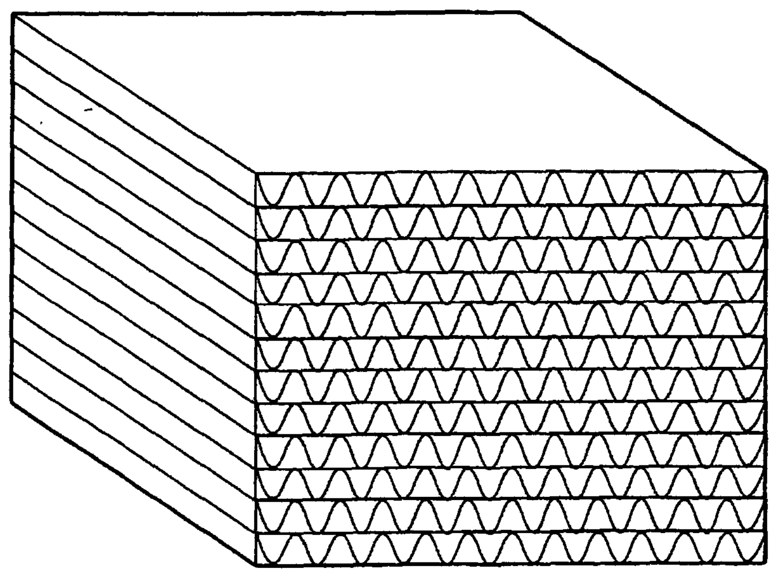

Fig. 7

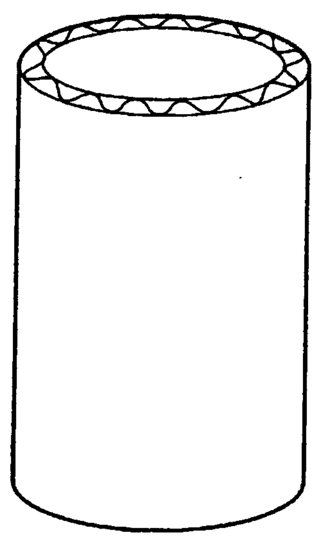

Fig. 8

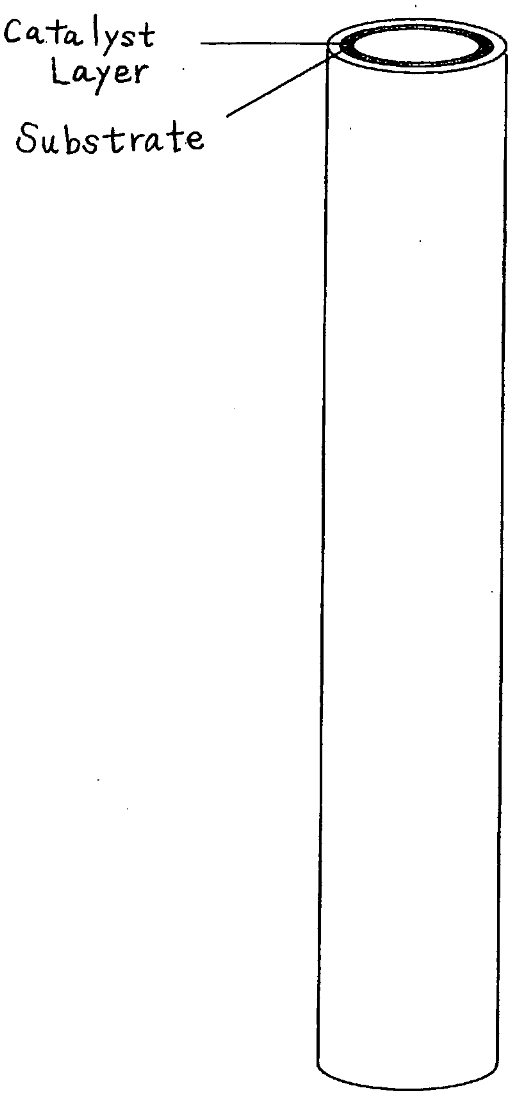

Fig. 9

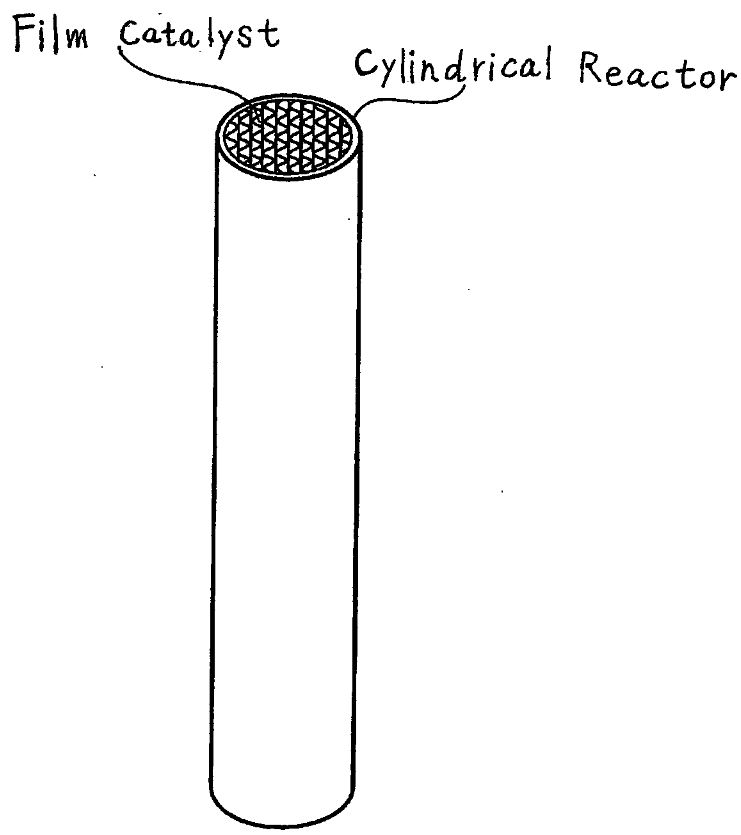

**INTERNATIONAL SEARCH REPORT**

International application No.
PCT/JP2007/070023

A. CLASSIFICATION OF SUBJECT MATTER
*B01J35/10*(2006.01)i, *B01J29/072*(2006.01)i

According to International Patent Classification (IPC) or to both national classification and IPC

B. FIELDS SEARCHED

Minimum documentation searched (classification system followed by classification symbols)
B01J35/10, B01J29/072

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2003-251201 A (Toyota Central Research and Development Laboratories, Inc.), 09 September, 2003 (09.09.03), Par. Nos. [0004], [0008], [0010], [0011]; examples; Fig. 1 (Family: none) | 1-22 |
| X | JP 58-51938 A (Rhone-Poulenc Specialites Chimiques), 26 March, 1983 (26.03.83), Examples 1 to 5 & US 4529718 A & EP 073703 A1 | 1-22 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>01 November, 2007 (01.11.07) | Date of mailing of the international search report<br>20 November, 2007 (20.11.07) |
| Name and mailing address of the ISA/<br>Japanese Patent Office | Authorized officer |
| Facsimile No. | Telephone No. |

Form PCT/ISA/210 (second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005035122 A

### Non-patent literature cited in the description

- Busshitsu no kinousei. Jikken Kagaku Kouza. Maruzen, vol. 12, 486